# EUROPEAN PATENT APPLICATION

(11) **EP 4 512 796 A1**
(43) Date of publication of application: **26.02.2025**
(21) Application number: 23791214.2
(22) Date of filing: 18.04.2023
(51) Int. Cl.: C07C 69/732, C07C 67/297, C07C 67/29, C07C 235/38, C07C 233/11, C07C 231/12, C07D 317/60, A61P 35/00, A61P 37/06, A61K 31/36, A61K 31/167

(54) **PHENYL ACRYLIC ACID COMPOUND, AND PREPARATION METHOD AND APPLICATION THEREOF**

(30) Priority: 20.04.2022 CN 202210419092
(71) Applicant: Longivitron (Suzhou) Biotechnology Co., Ltd, Suzhou, Jiangsu 215000 (CN)
(72) Inventor: LIU, Xiaoyu, Suzhou, Jiangsu 215000 (CN); CHEN, Xiaoguang, Suzhou, Jiangsu 215000 (CN); LI, Yan, Suzhou, Jiangsu 215000 (CN); LV, Shiliang, Suzhou, Jiangsu 215000 (CN); ZHAO, Yanshi, Suzhou, Jiangsu 215000 (CN)
(74) Representative: Buzzi, Notaro & Antonielli d'Oulx S.p.A.
(86) International application number: PCT/CN2023/088888
(87) International publication number: WO 2023/202559

(57) **Abstract**

The application provides a phenyl acrylic compound, and a preparation method and application thereof. The phenyl acrylic compound has a structure as shown in formula (I). The phenyl acrylic compound provided by the present application can be well combined with a plurality of target proteins, has good bioavailability, and can be used for preparing drugs for preventing or treating tumors, autoimmune diseases, inflammatory diseases, neurodegenerative diseases or anti-aging.

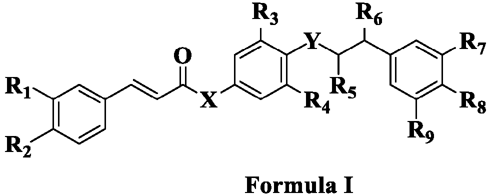

## Description

### TECHNICAL FIELD

The present application belongs to the field of medicines and, in particular, relates to a phenyl acrylic acid compound, a preparation method therefor and use thereof.

### BACKGROUND

The International Agency for Research on Cancer (IARC) of the World Health Organization released the latest estimates on the global burden of cancer for 2020. There were 19.29 million new cancer cases globally in 2020, including about 4.6 million new cancer cases in China. As China became the world's most populous country, the compound annual growth rate in the number of new cancer diagnoses in China was higher than the global average. By 2025, the number of new cancer cases is expected to reach 5.2 million in China, accounting for 26.9% of the number of new cases worldwide.

Medical data suggests that inflammation is one of the risk factors for tumors. For example, the number of patients with cervical cancer due to papillomavirus infection is growing; infection of the stomach with *Helicobacter pylori* tends to increase the risk of gastric cancer; chronic hepatitis may be directly responsible for causing liver cancer. There are other factors. For example, autoimmune enteropathy is closely related to colon cancer, and PM2.5 particulate matter in the air is also a vicious factor behind lung cancer. Tumors are defined as non-communicable diseases like heart diseases, chronic respiratory diseases or diabetes. Most of the tumors are chronic diseases, which progress relatively slowly. The correlation between inflammation and tumors was first proposed by Galenus 1800 years ago, and many studies have proven that persistent inflammation can cause lesions to progress from infections or autoimmune inflammation to tumors.

Phenyl acrylic acid compounds are widely found in active natural products, have a variety of biological activities such as antibacterial activity, antioxidant activity, anti-inflammatory activity and anti-tumor activity, and thus have broad research prospects. The applicant has long been engaged in the study of the structure optimization of phenyl acrylic acid natural products and the structure-function relationship with anti-tumor activity, treatment of autoimmune diseases and anti-inflammatory activity. The structure modification of the natural products can help to discover precursors with stronger tumor inhibition activity, stronger therapeutic activity for autoimmune diseases and inflammatory diseases, and lower toxicity than the original natural products, and such precursors can be further used as anti-tumor and anti-inflammatory immune drugs.

The potential anti-tumor targets of the phenyl acrylic acid derivatives designed in the present application are summarized below.

Caspase-3 is a caspase protein that interacts with caspase-8 and caspase-9. It is encoded by the CASP3 gene. Elevated levels of a fragment of Caspase-3, p17, in the bloodstream are a sign of a recent myocardial infarction. It is now being shown that caspase-3 may play an important role in the differentiation and apoptosis of embryonic and hematopoietic stem cells. It has been shown that the expression of caspase-3 in patients with head and neck cancer or breast cancer is abnormally high, so lowering the expression of caspase-3 is, to a certain extent, one of the ways of reducing the incidence of cancer.

The epidermal growth factor receptor (EGFR): Mutational activation of EGFR is an important factor leading to the abnormal biological activities of tumor cells. The T790M mutation is caused when a base pair in EGFR changes from cytosine (C) to thymine (T), that is, the threonine at site 790 in the tyrosine kinase function of EGFR is replaced by methionine. The mutation can reactivate the EGFR, thereby leading to resistance to tyrosine kinase inhibitors (TKIs). After such a mutated protein is docked with the phenyl acrylic acid natural products and the derivatives thereof by computer-aided design, it has been found that all resulting compounds scored highly, suggesting that phenyl acrylic acid derivatives can serve as candidates for the target.

Histone-lysine N-methyltransferase enzyme, EZH2: EZH2 is an enzyme encoded by the human EZH2 gene. It has been identified that two transcript variants transcribed from the aforementioned gene encode different isoforms; gene sequence alterations are fundamentally different from epigenetic modification abnormalities because once a DNA sequence is mutated, the gene is difficult to repair or the mutated gene product is difficult to eliminate. However, epigenetic modification abnormalities can potentially be reversed by inhibitors of chromatin-modification enzymes associated therewith. Therefore, it is important to clarify the mechanism of action of epigenetic modification enzymes in tumor cells, thereby providing appropriate therapeutic means to prevent epigenetic modification mutations. Currently, EZH2 inhibitor drugs have not been commercially available yet, and a total of five drugs are in clinical phase I/II.

Histone deacetylase (HDAC): HDAC is a class of proteases that play an important role in structure modification of chromosomes and regulation of gene expression. In general, histone acetylation facilitates the dissociation of DNA from histone octamers, and the structure of nucleosomes is relaxed, thereby enabling various transcription factors and co-regulators to bind specifically to DNA binding sites and activating gene transcription. In the nucleus, histone acetylation and histone deacetylation are in dynamic equilibrium and are jointly regulated by histone acetyltransferases (HATs) and histone deacetylases (HDACs).

Src kinase: Src kinases, a type of non-receptor protein kinases, are widely found in cancer cells and play an important role in various processes of cell growth and proliferation, such as gene transcription, cell differentiation, migration, angiogenesis, prevention of apoptosis, etc. The study of Src inhibitors has become a hot spot in the research of anti-tumor drugs. Currently, a series of Src inhibitors are undergoing clinical studies.

Cancer immunotherapy is an increasingly effective strategy to treat cancer. T cells play a key role in immunotherapy, and the numerous immune checkpoints are treasures to be discovered. Since the efficacy of CTLA-4 and PD-1/PD-L1 monoclonal antibodies has been affirmed, there has been intense competition on the track of monoclonal antibodies. Therefore, more attention should be paid to other immune checkpoints and targets whose functions determine the efficacy of immunotherapy.

The survival and development of T cells are affected by T cell receptor (TCR) signals, while the TCR signaling pathway is dependent on Src family kinases (SFK). Lck is an important member of SFK and is expressed throughout most of the life cycle of T cells. Moreover, Lck plays an important role in activating the TCR signaling pathway to activate T cells. CSK is a key regulator for SFK and can inactivate Lck by phosphorylation of Lck (Tyr505), and then inhibits the activation of T cells via TCR. Therefore, CSK and p-Lck (Tyr505) may be effective targets for future immunoregulation therapy.

CD73 is a 5-nucleotidase and can hydrolyze extracellular adenosine monophosphate (AMP) to adenosine. Adenosine, as a powerful immunosuppressive molecule, can inhibit the activation of CD8-positive T cells and thus help cancer cells to escape from the "hunting" of T cells. Tumor-infiltrating natural killer (NK) cells upregulate the expression of CD73, and the incidence of these CD73+NK cells correlates with the tumor size in breast cancer patients. It is supported by research that tumors can hijack NK cells for immune evasion and that CD73 expression defines an inducible NK cell population and has immunoregulation properties in the tumor microenvironment.

KIR: Killer-cell immunoglobulin-like receptors (KIRs) are expressed on the cell membrane of NK cells and a minority of T cells and can specifically recognize MHC-I molecules on the cell surface and then exert immunoregulation functions.

LAG-3: Lymphocyte-activation gene 3 (LAG-3) is a receptor for negative immunoregulation molecules. LAG-3 is distributed in activated T cells, NK cells and dendritic cells, can bind to MHC-II molecules, has the function of maintaining the stability of the internal environment and participating in immunoregulation, and is closely related to the occurrence and development of tumors.

4-1BB, also known as CD137, is a member of the tumor necrosis factor (TNF) receptor family and is expressed on the surface of activated T cells. 4-1BB is an inducible T-cell surface receptor. 4-1BB, as well as its ligand, is another important co-stimulatory molecule in addition to the CD28/B7 co-stimulatory signaling pathway.

ACAT1 belongs to the specific thiolase superfamily and is known as acetoacetyl-CoA thiolase or acetyl-CoA acetyltransferase (ACAT). It has been reported that the expression of ACAT1 in tumor cells is usually aberrant, and ACAT1 plays a crucial role in the occurrence and development of tumors. The mechanism of up-regulation of ACAT1 activity in different human cancer cells is worth studying. High expression of ACAT1 decreases its overall survival. The reuse of the ketone bodies generated from ACAT1 overexpression in MDA-MB-231 human breast cancer cells drives the progression and metastasis of tumors, and thus, the study of phenyl acrylic acid derivatives targeting ACAT1 may lead to the discovery of novel anti-tumor drugs.

The antioxidant and anti-inflammatory immunity of the phenyl acrylic acid compounds are as follows.

The phenyl acrylic acid structure is widely found in polyphenolic secondary metabolites of plants and has strong antioxidant and anti-inflammatory properties. The phenyl acrylic acid structure is capable of chelating metal ions and scavenging free radicals [superoxide anion (O₂⁻), hydrogen peroxide (H₂O₂), hydroxyl radical (•OH), hypochlorous acid (HOCl), peroxynitrite anion (ONOO-), and nitric oxide (NO)]. The compounds having a phenyl acrylic acid structure have strong antioxidant activity. The activity of such compounds for scavenging 1,1-diphenyl-2-picrylhydrazyl (DPPH) free radicals is 2-3 times that of vitamins C and E, and its activity for scavenging superoxide anion free radicals is 10-30 times that of vitamins C and E.

In terms of anti-inflammatory action, the phenyl acrylic acid compounds have anti-inflammatory activity on a Carrageenan-induce raw paw edema model. Its mechanism is as follows: the production of interleukin-8 (IL-8) can be inhibited by scavenging intracellular ROS, inhibiting p38 cascade phosphorylation and up-regulating nuclear factor kappa-B (NF-κB) signaling pathway, thereby exerting anti-inflammatory effects.

However, in a mouse model of dextran sulfate-induced ulcerative colitis, inflammatory responses of the tissues are reduced by modulating the kinase signaling pathway at the amino-terminal of the mitogen-activated protein kinase/ERK/c-Jun. Therefore, the phenyl acrylic acid compounds can be called a new therapeutic agent for the treatment of rheumatoid arthritis, and its mechanism is that the B-cell activating factor expression mediated through the NF-κB pathway is inhibited by reducing the DNA-binding capacity of NF-κB to the promoter region of the B-cell activating factor. The phenyl acrylic acid compounds also have a strong protective effect on liver function and can effectively reduce the production of TNF-α, IL-6 and IL-1β inflammatory factors due to CCl₄-induced acute liver injury. Its mechanism is that the liver is protected from acute liver injury through the antioxidant action mediated by nuclear factor E-2-related factor 2 (Nrf2) and the inhibition of activation of Nod-like receptor family pyrin domain containing 3 inflammasomes. Such compounds can also reduce methotrexate (MTX)-induced hepatotoxicity by increasing B-cell lymphoma-2 (Bcl2) expression and inhibiting the inflammatory responses mediated by cyclooxygenase-2, inducible nitric oxide synthase, Bcl2 associated X (Bax), caspases 3 and caspases 9.

Pathological studies of neurodegenerative diseases including Alzheimer's disease and Parkinson's disease have shown that chronic oxidative stress and inflammatory responses lead to damage to the neuron. The phenyl acrylic acid compounds have been found to have a good neuroprotective effect due to their strong antioxidant and anti-inflammatory effects. Several clinical and preclinical studies have shown that phenyl acrylic acid natural products exhibit good therapeutic effects against Alzheimer's disease and Parkinson's disease. The phenyl acrylic acid compounds can alleviate memory loss and prevent hippocampal cells from death after transient forebrain ischemia. Its mechanism is that spatial memory is improved by increasing the expression of Bcl2, superoxide dismutase 2, and the platelet endothelial cell adhesion molecule, CD31, and decreasing the expression of endothelin-1, thereby preventing CA1 pyramidal cells from death after occlusion of bilateral common carotid arteries. The phenyl acrylic acid compounds can prevent saturated free fatty acid (FFA)-induced lipotoxicity by activating SIRT1-regulated mitochondrial function. Its mechanism is that oxidative stress and mitochondrial dysfunction are attenuated by reducing the production of ROS and increasing mitochondrial mass and mitochondrial membrane potential, and mitochondria-mediated caspase-dependent apoptosis is reduced by significantly decreasing the expression of the pro-apoptosis protein Bax.

Although phenyl acrylic acid derivatives have been proven to have a wide range of activities as described above, their exact mechanism of action and structure-function relationships still need to be further studied, and it is necessary to synthesize their structural derivatives and study their drug mode of action as well as the diseases that they can be used to treat especially inflammation-related diseases, tumors, autoimmune diseases, neurodegenerative diseases and aging.

### SUMMARY

The present application provides a phenyl acrylic acid compound, a preparation method therefor and use thereof. The phenyl acrylic compound provided by the present application can be well combined with a plurality of target proteins, has good bioavailability, and can be used for preparing drugs for preventing or treating tumors, autoimmune diseases, inflammatory diseases, neurodegenerative diseases or aging.

In a first aspect, the present application provides a phenyl acrylic acid compound. The phenyl acrylic acid compound has a structure represented by Formula I: 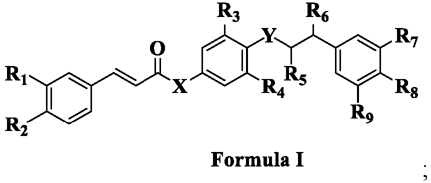
wherein R₁, R₂, R₃, R₄, R₇, R₈ and R₉ are each independently selected from any one of hydrogen, halogen, hydroxyl, dimethylamino, cyano, nitro, methylamino, methylsulfonyl, dimethylsulfamoyl, amino, carboxyl, C1-C6 (which, for example, may be C1, C2, C3, C4, C5 or C6) alkoxycarbonyl, C1-C6 (which, for example, may be C1, C2, C3, C4, C5 or C6) alkylcarbonyloxy, C1-C6 (which, for example, may be C1, C2, C3, C4, C5 or C6) alkyl, C1-C6 (which, for example, may be C1, C2, C3, C4, C5 or C6) alkoxy, C1-C6 (which, for example, may be C1, C2, C3, C4, C5 or C6) alkoxymethyleneoxy, C1-C6 (which, for example, may be C1, C2, C3, C4, C5 or C6) alkanoyl, C1-C6 (which, for example, may be C1, C2, C3, C4, C5 or C6) trihaloalkyl, or C1-C6 (which, for example, may be C1, C2, C3, C4, C5 or C6) trihaloalkoxy; or, R₁, R₂, R₇, R₈ and R₉ are each independently selected from -O(CH₂)ₙO- and joined to substituted phenyl to form a ring, wherein n is selected from 1, 2 or 3;
R₅ is selected from any one of hydrogen, amino, C1-C6 (which, for example, may be C1, C2, C3, C4, C5 or C6) alkyl, C1-C6 (which, for example, may be C1, C2, C3, C4, C5 or C6) alkoxymethyl, or C1-C6 (which, for example, may be C1, C2, C3, C4, C5 or C6) alkylamino;
R₆ is selected from any one of hydrogen, hydroxyl, amino, carbonyl, C1-C6 (which, for example, may be C1, C2, C3, C4, C5 or C6) alkylamino, C1-C6 (which, for example, may be C1, C2, C3, C4, C5 or C6) alkylcarbonyloxy, or C1-C6 (which, for example, may be C1, C2, C3, C4, C5 or C6) alkoxycarbonyl;
X is selected from O or NH; and
Y is selected from any one of O, NH, S, sulfoxide or sulfone.

In the present application, "trihalo" in C1-C6 trihaloalkyl or C1-C6 trihaloalkoxy means containing three halogens. C1-C6 trihaloalkyl includes F₃ C1-C6 alkyl, Br₃ C1-C6 alkyl, Cl₃ C1-C6 alkyl, I₃ C1-C6 alkyl, F₂Br C1-C6 alkyl, F₂Cl C1-C6 alkyl, F₂I C1-C6 alkyl, FBr₂ C1-C6 alkyl, FCl₂ C1-C6 alkyl, FI₂ C1-C6 alkyl, ClBr₂ C1-C6 alkyl, ICl₂ C1-C6 alkyl, and ClI₂ C1-C6 alkyl; and C1-C6 trihaloalkoxy includes F₃ C1-C6 alkoxy, Br₃ C1-C6 alkoxy, Cl₃ C1-C6 alkoxy, I₃ C1-C6 alkoxy, F₂Br C1-C6 alkoxy, F₂Cl C1-C6 alkoxy, F₂I C1-C6 alkoxy, FBr₂ C1-C6 alkoxy, FCl₂ C1-C6 alkoxy, FI₂ C1-C6 alkyl, ClBr₂ C1-C6 alkoxy, ICl₂ C1-C6 alkoxy, and ClI₂ C1-C6 alkoxy.

Preferably, C1-C6 trihaloalkyl includes F₃ C1-C4 alkyl, Br₃ C1-C4 alkyl, Cl₃ C1-C4 alkyl, I₃ C1-C4 alkyl, F₂Br C1-C4 alkyl, F₂Cl C1-C4 alkyl, F₂I C1-C4 alkyl, FBr₂ C1-C4 alkyl, FCl₂ C1-C4 alkyl, FI₂ C1-C4 alkyl, ClBr₂ C1-C4 alkyl, ICl₂ C1-C4 alkyl, and ClI₂ C1-C4 alkyl; and C1-C6 trihaloalkoxy includes F₃ C1-C4 alkoxy, Br₃ C1-C4 alkoxy, Cl₃ C1-C4 alkoxy, I₃ C1-C4 alkoxy, F₂Br C1-C4 alkoxy, F₂Cl C1-C4 alkoxy, F₂I C1-C4 alkoxy, FBr₂ C1-C4 alkoxy, FCl₂ C1-C4 alkoxy, FI₂ C1-C4 alkyl, ClBr₂ C1-C4 alkoxy, ICl₂ C₁₋₄ alkoxy, and ClI₂ C1-C4 alkoxy.

More preferably, C1-C6 trihaloalkyl includes F₃C, F₃CCH₂, Br₃C, Br₃CCH₂, Cl₃C, Cl₃CCH₂, I₃C, I₃CCH₂, F₂BrC, F₂BrCCH₂, F₂ClC, F₂CICCH₂, F₂IC, F₂ICCH₂, FBr₂C, FBr₂CCH₂, FCl₂C, FCl₂CCH₂, FI₂C, FI₂CCH₂, ClBr₂C, ClBr₂CCH₂, ICl₂C, ICl₂CCH₂, ClI₂C, and ClI₂CCH₂; and C1-C6 trihaloalkoxy includes F₃CO, F₃CCH₂O, Br₃CO, Br₃CCH₂O, Cl₃CO, Cl₃CCH₂O, I₃CO, I₃CCH₂O, F₂BrCO, F₂BrCCH₂O, F₂ClCO, F₂ClCCH₂O, F₂ICO, F₂ICCH₂O, FBr₂CO, FBr₂CCH₂O, FCl₂CO, FCl₂CCH₂O, FI₂CO, FI₂CCH₂O, ClBr₂CO, ClBr₂CCH₂O, ICl₂CO, ICl₂CCH₂O, ClI₂CO, and ClI₂CCH₂O.

In the present application, the phenyl acrylic acid compound is selected from any one of Formula II to Formula V: 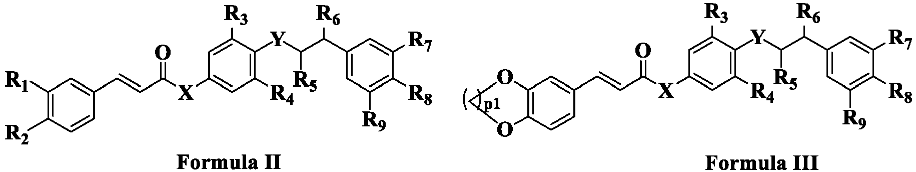 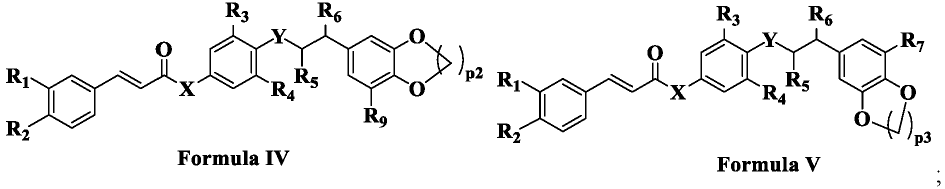
wherein R₁ and R₂ are each independently selected from any one of hydrogen, fluorine, chlorine, bromine, hydroxyl, dimethylamino, cyano, nitro, methylamino, methylsulfonyl, dimethylsulfamoyl, amino, carboxyl, C1-C4 (which, for example, may be C1, C2, C3 or C4) alkoxycarbonyl, C1-C4 (which, for example, may be C1, C2, C3 or C4) alkylcarbonyloxy, C1-C4 (which, for example, may be C1, C2, C3 or C4) alkyl, C1-C4 (which, for example, may be C1, C2, C3 or C4) alkoxy, C1-C4 (which, for example, may be C1, C2, C3 or C4) alkoxymethyleneoxy, C1-C4 (which, for example, may be C1, C2, C3 or C4) trihaloalkyl, or C1-C4 (which, for example, may be C1, C2, C3 or C4) trihaloalkoxy;
R₃ and R₄ are each independently selected from any one of hydrogen, fluorine, chlorine, bromine, hydroxyl, dimethylamino, cyano, nitro, methylamino, methylsulfonyl, dimethylsulfamoyl, amino, carboxyl, C1-C4 (which, for example, may be C1, C2, C3 or C4) alkoxycarbonyl, C1-C4 (which, for example, may be C1, C2, C3 or C4) alkylcarbonyloxy, C1-C4 (which, for example, may be C1, C2, C3 or C4) alkyl, C1-C4 (which, for example, may be C1, C2, C3 or C4) alkoxy, C1-C4 (which, for example, may be C1, C2, C3 or C4) trihaloalkyl, or C1-C4 (which, for example, may be C1, C2, C3 or C4) trihaloalkoxy;
R₅ is selected from any one of hydrogen, amino, C1-C4 (which, for example, may be C1, C2, C3 or C4) alkyl, C1-C4 (which, for example, may be C1, C2, C3 or C4) alkoxymethyl or C1-C4 (which, for example, may be C1, C2, C3 or C4) alkylamino;
R₆ is selected from any one of hydrogen, hydroxyl, amino, carbonyl, C1-C4 (which, for example, may be C1, C2, C3 or C4) alkylamino, C1-C4 (which, for example, may be C1, C2, C3 or C4) alkylcarbonyloxy or C1-C4 (which, for example, may be C1, C2, C3 or C4) alkoxycarbonyl;
R₇, R₈ and R₉ are each independently selected from any one of hydrogen, fluorine, chlorine, bromine, hydroxyl, dimethylamino, cyano, nitro, methylamino, methylsulfonyl, dimethylsulfamoyl, amino, carboxyl, C1-C4 (which, for example, may be C1, C2, C3 or C4) alkyl, C1-C4 (which, for example, may be C1, C2, C3 or C4) alkoxy, C1-C4 (which, for example, may be C1, C2, C3 or C4) alkoxycarbonyl, C1-C4 (which, for example, may be C1, C2, C3 or C4) alkylcarbonyloxy, C1-C3 (which, for example, may be C1, C2 or C3) alkanoyl, C1-C4 (which, for example, may be C1, C2, C3 or C4) alkoxymethyleneoxy, C1-C4 (which, for example, may be C1, C2, C3 or C4) trihaloalkyl or C1-C4 (which, for example, may be C1, C2, C3 or C4) trihaloalkoxy;
X is selected from O or NH;
Y is selected from any one of O, NH, S, sulfoxide or sulfone; and
p1, p2 and p3 are each independently selected from 1, 2 or 3.

In the present application, R₁ and R₂ are each independently selected from any one of hydrogen, fluorine, chlorine, bromine, hydroxyl, dimethylamino, cyano, nitro, methoxycarbonyl, ethoxycarbonyl, methylamino, methylsulfonyl, dimethylsulfamoyl, amino, methyl, ethyl, methoxy, ethoxy, C1-C2 (which, for example, may be C1 or C2) alkylcarbonyloxy, C1-C2 (which, for example, may be C1 or C2) alkoxymethyleneoxy, C1-C2 (which, for example, may be C1 or C2) trihaloalkyl or C1-C2 (which, for example, may be C1 or C2) trihaloalkoxy;
R₃ and R₄ are each independently selected from any one of hydrogen, fluorine, chlorine, bromine, hydroxyl, dimethylamino, cyano, nitro, methoxycarbonyl, ethoxycarbonyl, methylamino, methylsulfonyl, dimethylsulfamoyl, amino, methyl, ethyl, methoxy, ethoxy, C1-C2 (which, for example, may be C1 or C2) alkylcarbonyloxy, C1-C2 (which, for example, may be C1 or C2) trihaloalkyl or C1-C2 (which, for example, may be C1 or C2) trihaloalkoxy;
R₅ is selected from any one of hydrogen, amino, methyl, ethyl, C1-C2 (which, for example, may be C1 or C2) alkoxymethyl or C1-C2 (which, for example, may be C1 or C2) alkylamino;
R₆ is selected from any one of hydrogen, amino, hydroxyl, carbonyl, C1-C2 (which, for example, may be C1 or C2) alkylamino, C1-C2 (which, for example, may be C1 or C2) alkylcarbonyloxy, or C1-C2 (which, for example, may be C1 or C2) alkoxycarbonyl;
R₇, R₈ and R₉ are each independently selected from any one of hydrogen, fluorine, chlorine, bromine, hydroxyl, dimethylamino, cyano, nitro, methylamino, methylsulfonyl, dimethylsulfamoyl, amino, carboxyl, methyl, ethyl, methoxy, ethoxy, methoxycarbonyl, ethoxycarbonyl, formyl, acetyl, propionyl, butyryl, pentanoyl, isobutyryl, 2-methylbutanoyl, C1-C2 (which, for example, may be C1 or C2) alkoxymethyleneoxy, C1-C2 (which, for example, may be C1 or C2) trihaloalkyl or C1-C2 (which, for example, may be C1 or C2) trihaloalkoxy;
X is selected from O or NH;
Y is selected from any one of O, NH, S, sulfoxide or sulfone; and
p1, p2 and p3 are each independently selected from 1 or 2.

In the present, R₁ and R₂ are each independently selected from any one of hydrogen, fluorine, chlorine, bromine, hydroxyl, dimethylamino, cyano, nitro, methoxycarbonyl, methylamino, methylsulfonyl, dimethylsulfamoyl, amino, methyl, ethyl, methoxy, ethoxy, trifluoromethyl, trifluoromethoxy or methoxymethyleneoxy;
R₃ and R₄ are each independently selected from any one of hydrogen, fluorine, chlorine, bromine, hydroxyl, dimethylamino, cyano, nitro, methoxycarbonyl, methylamino, methylsulfonyl, dimethylsulfamoyl, amino, methyl, ethyl, methoxy, ethoxy, trifluoromethyl or trifluoromethoxy;
R₅ is selected from any one of hydrogen, methyl, ethyl, methoxymethyl or amino;
R₆ is selected from any one of hydrogen, hydroxyl, amino, carbonyl or C1-C2 (which, for example, may be C1 or C2) alkylcarbonyloxy;
R₇, R₈ and R₉ are each independently selected from any one of hydrogen, fluorine, chlorine, bromine, hydroxyl, dimethylamino, cyano, nitro, methylamino, methylsulfonyl, dimethylsulfamoyl, amino, carboxyl, methyl, ethyl, methoxy, ethoxy, trifluoromethyl, trifluoromethoxy, methoxycarbonyl, formyl, acetyl, propionyl, butyryl, pentanoyl, isobutyryl or methoxymethyleneoxy;
X is selected from O or NH;
Y is selected from any one or a combination of at least two of O, NH, S, sulfoxide or sulfone; and
p1, p2 and p3 are each independently selected from 1.

In the present application, the phenyl acrylic acid compound is selected from any one of M1 to M31: 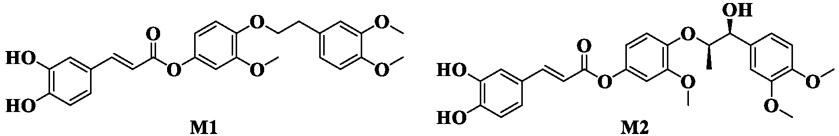 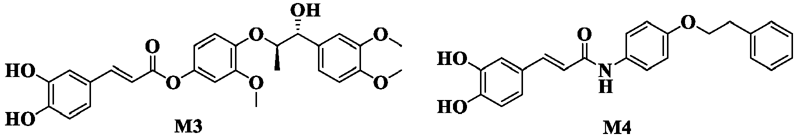 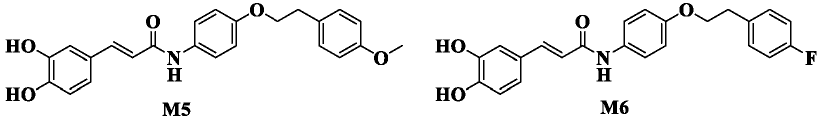 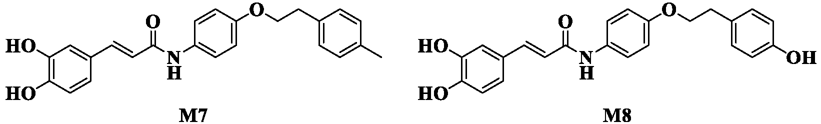 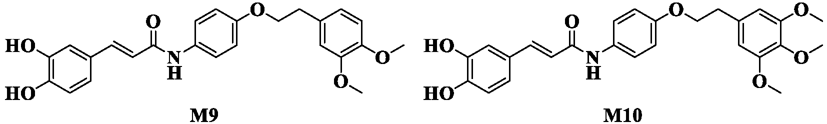 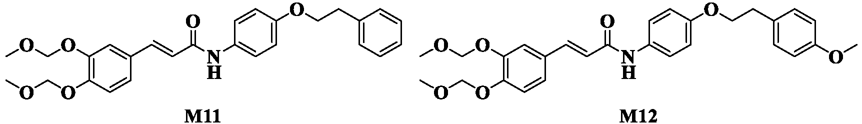 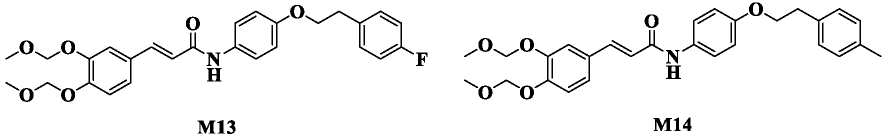 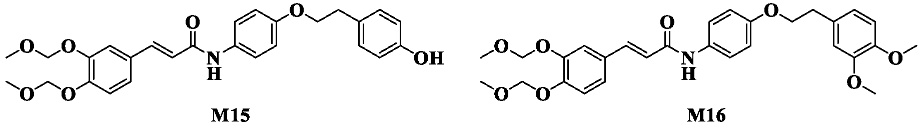 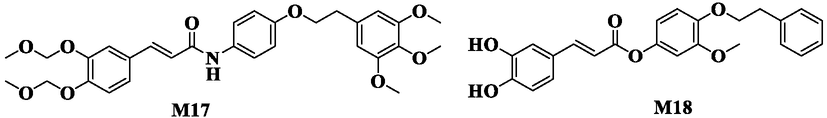 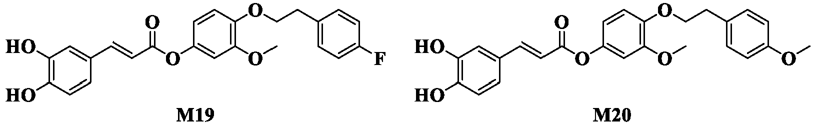 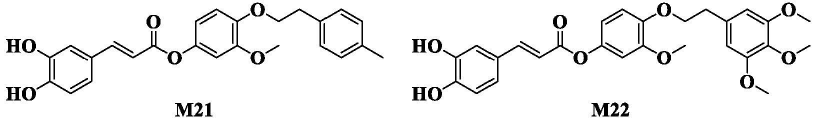 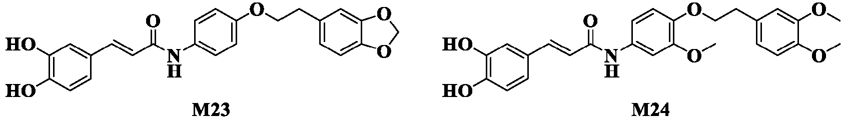 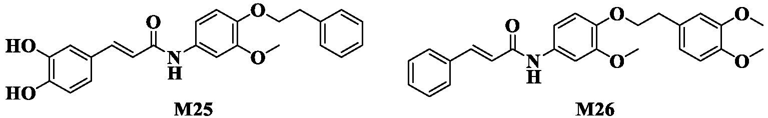 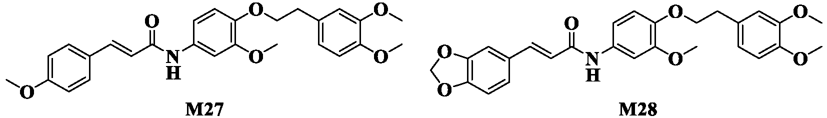 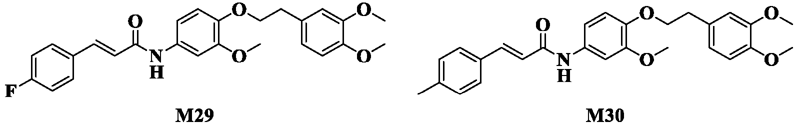 or

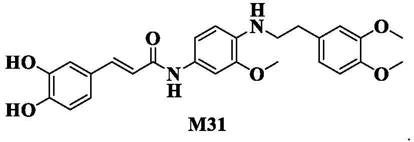

In a second aspect, the present application provides a tautomer, enantiomer or diastereomer of the phenyl acrylic acid compound described in the first aspect, or a pharmaceutically acceptable salt thereof.

Preferably, the pharmaceutically acceptable salt includes any one or a combination of at least two of hydrochloride, hydrobromide, phosphate, sulphate, methanesulfonate, p-toluenesulfonate, acetate, trifluoroacetate, salicylate, amino acid salt, 2-O-β-D-glucopyranosyl-L-ascorbic acid salt, maleate, tartrate, fumarate, citrate, lactate, a sodium salt, a potassium salt, a calcium salt, a magnesium salt, a lithium salt, an ammonium salt, or a salt of an organic base capable of providing a physiologically acceptable cation.

Preferably, the salt of an organic base capable of providing a physiologically acceptable cation includes any one or a combination of at least two of a methylamine salt, a dimethylamine salt, a trimethylamine salt, a piperidine salt, a morpholine salt or a tris(2-hydroxyethyl)amine salt.

In the present application, the term "halogen" refers to fluorine, chlorine, bromine or iodine.

According to the present application, the compound of Formula I may exist in the form of an isomer, and the configuration of the carbon of the compound of Formula I connecting the groups R₅ and R₆ groups may be an R or S configuration.

The present application includes all possible stereoisomers as well as mixtures of two or more isomers.

If cis/trans isomers exist, the present application covers cis and trans forms and the mixtures thereof, and a single isomer, if required, may be isolated according to conventional methods or prepared by stereoselective synthesis.

All salts in the present application may be prepared by conventional methods. Furthermore, polymorphic or eutectic products may be produced under different crystallization conditions during the preparation of the solvates of the compound represented by Formula I and the salts thereof.

In a third aspect, the present application provides a preparation method for the phenyl acrylic acid compound described in the first aspect. The preparation method includes the following steps:
(1) carrying out a condensation reaction on a compound represented by Formula a and a compound represented by Formula b to obtain a compound represented by Formula c, wherein the reaction formula is shown below: 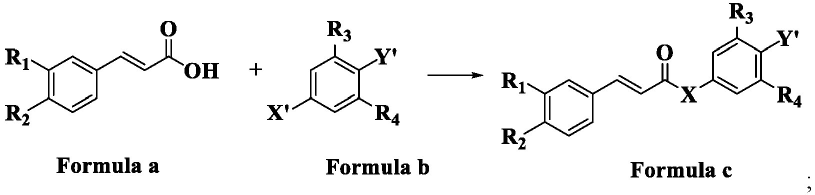
(2) carrying out deprotection on the compound represented by Formula c to obtain a compound represented by Formula d, wherein the reaction formula is shown below: 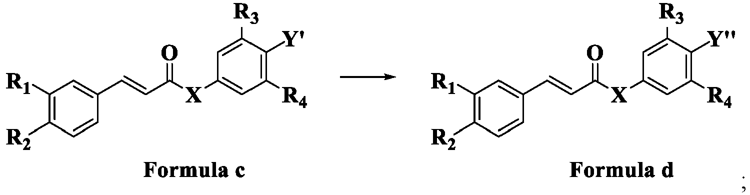
(3) carrying out a substitution reaction on the compound represented by Formula d and a compound represented by Formula e to obtain a compound represented by Formula I, wherein the reaction formula is shown below: 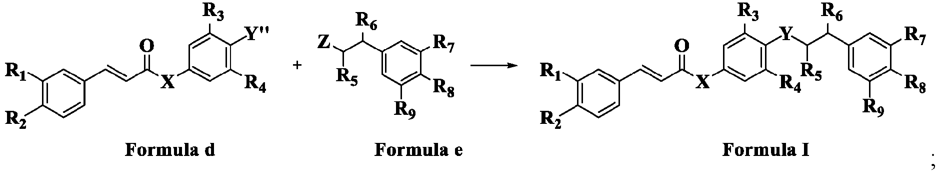 or the preparation method includes the following step: carrying out a condensation reaction on a compound represented by Formula f and a compound represented by Formula g to obtain a compound represented by Formula I, wherein the reaction formula is shown below: 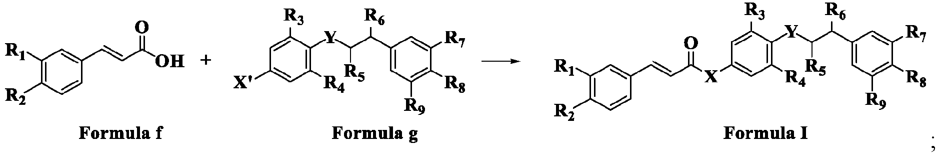
   wherein X' is selected from OH or NH₂;
   Y' is selected from any one of *tert*-butyldimethylsilyloxy, *tert*-butyldiphenylsilyloxy, trimethylsilyloxy, triethylsilyloxy, benzyloxy, p-methoxybenzyloxy, methoxymethyleneoxy, benzyloxycarbonyloxy or tert-butoxycarbonyloxy;
   Y" is selected from any one of OH, NH₂ or SH; and
   Z is selected from any one of hydroxyl, chlorine, bromine, iodine, p-toluenesulfonyl or methylsulfonyl.

In the present application, in step (1), the condensation reaction is carried out under an alkaline condition.

Preferably, in step (1), the condensation reaction is carried out in the presence of a condensating agent, and the condensating agent includes EDCI and/or DMAP.

Preferably, in step (1), the condensation reaction is carried out at a temperature of 0 °C to 30 °C (for example, 0 °C, 5 °C, 10 °C, 15 °C, 20 °C, 25 °C, 30 °C, etc.) for 5 hours to 15 hours (for example, 5 hours, 7 hours, 9 hours, 11 hours, 13 hours, 15 hours, etc.).

Preferably, in step (1), a molar ratio of the compound represented by Formula a to the compound represented by Formula b is 1:(0.8-1.5);
wherein "0.8-1.5" may be 0.8, 0.9, 1, 1.1, 1.2, 1.3, 1.4, 1.5, etc.

Preferably, in step (2), the deprotection is carried out via a hydrolysis reaction or a hydrogenation reaction.

Preferably, in step (2), the deprotection is carried out via a hydrolysis reaction, and the hydrolysis reaction is carried out under an acidic condition or an alkaline condition.

Preferably, in step (3), the substitution reaction is carried out under an alkaline condition.

Preferably, in step (3), the substitution reaction is carried out at a temperature of 60 °C to 100 °C (for example, 60 °C, 65°C, 70 °C, 75 °C, 80 °C, 85 °C, 90 °C, 95 °C, 100 °C, etc.) for 2 hours to 6 hours (for example, 2 hours, 2.5 hours, 3 hours, 3.5 hours, 4 hours, 4.5 hours, 5 hours, 5.5 hours, 6 hours, etc.).

Preferably, in step (3), a molar ratio of the compound represented by Formula d to the compound represented by Formula e is 1:(1-2);
wherein "1-2" may be 1, 1.1, 1.2, 1.3, 1.4, 1.5, 1.6, 1.7, 1.8, 1.9, 2, etc.

Preferably, the condensation reaction is carried out on the compound represented by Formula f and the compound represented by Formula g under an alkaline condition.

Preferably, the condensation reaction is carried out on the compound represented by Formula f and the compound represented by Formula g in the presence of a condensating agent, and the condensating agent includes EDCI and/or DMAP.

Preferably, a molar ratio of the compound represented by Formula f to the compound represented by Formula g is 1:(0.8-1.5);
wherein "0.8-1.5" may be 0.8, 1, 1.1, 1.2, 1.3, 1.4, 1.5, etc.

In a fourth aspect, the present application provides a pharmaceutical composition. The pharmaceutical composition includes an active ingredient and a pharmacodynamically acceptable carrier, wherein the active ingredient includes the phenyl acrylic acid compound described in the first aspect or the tautomer, enantiomer or diastereomer of the phenyl acrylic acid compound or the pharmaceutically acceptable salt thereof described in the second aspect.

Preferably, a mass percentage of the active ingredient in the pharmaceutical composition is 0.1% to 95%, for example, 0.1, 1, 10, 20, 30, 40, 50, 60, 70, 80, 90, 95, etc.

The pharmaceutical composition provided by the present application may be prepared according to methods well-known in the art. An active ingredient may be combined with one or more pharmaceutically acceptable solid or liquid excipients and/or excipients and then prepared into any dosage form suitable for humans or animals.

The phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the present application may be administered in a unit-dose form and by enteral administration or parenteral administration, for example, orally, intravenously, intramuscularly, subcutaneously, nasally, oromucosally, ocularly, pulmonarily and respiratorily, transdermally, intravaginally or intrarectally.

A dosage form may be a liquid dosage form, a solid dosage form or a semi-solid dosage form. The liquid dosage form may be solutions (including true solutions and colloidal solutions), emulsions (including o/w emulsions, w/o emulsions and multiple emulsions), suspensions, injections (including aqueous injections, powder injections and infusions), eye drops, nasal drops, lotions, and liniments. The solid dosage form may be tablets (including conventional tablets, enteric-coated tablets, buccal tablets, dispersible tablets, chewable tablets, effervescent tablets and orally disintegrating tablets), capsules (including hard capsules, soft capsules and enteric-coated capsules), granules, dispersions, micropellets, drops, suppositories, films, patches, (powder) aerosols, and sprays. The semi-solid dosage form may be ointments, gels, and pastes.

The phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the present application may be prepared into conventional formulations or may be prepared into sustained release formulations, controlled release formulations, targeting formulations, and various particulate delivery systems.

To prepare the phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the present application into tablets, various excipients well known in the art, including diluents, binding agents, wetting agents, disintegrating agents, lubricants, and glidants. The diluent may be starch, dextrin, sucrose, glucose, lactose, mannitol, sorbitol, xylitol, microcrystalline cellulose, calcium sulphate, calcium hydrogen phosphate, and calcium carbonate. The wetting agent may be water, ethanol, and isopropanol. The binding agent may be starch slurry, dextrin, molasses, honey, glucose solution, microcrystalline cellulose, acacia mucilage, gelatin solution, sodium carboxymethyl cellulose, methylcellulose, hydroxypropyl methylcellulose, ethylcellulose, acrylic resin, carbomer, polyvinylpyrrolidone, and polyethylene glycol. The disintegrating agent may be dry starch, microcrystalline cellulose, low-substituted hydroxypropyl cellulose, cross-linked polyvinylpyrrolidone, cross-linked sodium carboxymethylcellulose, sodium carboxymethyl starch, sodium bicarbonate with citric acid, polyoxyethylene sorbitol fatty acid ester, and sodium dodecyl sulfate. The lubricant and the glidant may be talcum powder, silicon dioxide, stearate, tartaric acid, liquid paraffin, and polyethylene glycol.

The tablets may further be made into coated tablets, such as sugar-coated tablets, film-coated tablets, enteric-coated tablets, or bilayer tablets and multilayer tablets.

To prepare the phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect into capsules, the phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect may be mixed with a diluent and a glidant, and the resulting mixture may be directly loaded in a hard capsule or a soft capsule. The phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect may also be prepared into granules or micropellets with a diluent, a binding agent and a disintegrating agent, and the resulting granules or micropellets are loaded in a hard capsule or a soft capsule. The diluents, binding agents, wetting agents, disintegrating agents, and glidants used in the preparation of tablets may also be used in the preparation of capsules of the compound of the present application.

To prepare the phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the present application into injections, water, ethanol, isopropanol, propylene glycol or a mixture thereof may be used as a solvent, and appropriate quantities of solubilizer, cosolvent, pH adjusting agent and osmotic pressure regulator, which are commonly used in the art, may be added. The solubilizer or cosolvent may be poloxamer, lecithin, and hydroxypropyl-beta-cyclodextrin. The pH-adjusting agent may be phosphate, acetate, hydrochloric acid, and sodium hydroxide. The osmotic pressure regulator may be sodium chloride, mannitol, glucose, phosphate, and acetate. If a lyophilized powder injection is required to be prepared, mannitol, glucose and the like may also be added as a support agent.

In addition, colorants, preservatives, spices, corrigent or other additives may also be added to the pharmaceutical preparation, if required.

To achieve the purpose of administration and enhance the therapeutic effect, the drug or the pharmaceutical composition of the present application may be administered by any well-known method of administration.

In a fifth aspect, the present provides the use of the phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the preparation of a drug for preventing or treating a tumor, an autoimmune disease, an inflammatory disease, a neurodegenerative disease or aging.

Preferably, the tumor is selected from any one or a combination of at least two of glioma, melanoma, gastric cancer, lung cancer, breast cancer, kidney cancer, liver cancer, oral epithelial carcinoma, head and neck tumor, cervical cancer, ovarian cancer, pancreatic cancer, prostate cancer, colon cancer, rectal adenocarcinoma, leukemia or lymphoma.

Preferably, the autoimmune disease includes any one or a combination of at least two of rheumatoid arthritis, systemic lupus erythematosus, ulcerative colitis, psoriasis, dermatitis or lateral sclerosis of the spinal cord.

Preferably, the inflammatory disease includes any one or a combination of at least two of polyarteritis, phlebitis or reflux esophagitis.

Preferably, the neurodegenerative disease includes senile dementia and/or Parkinson's disease.

The phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the present application may be administered in a wide range of doses, depending on the nature and severity of the disease to be prevented or treated, the individual condition of the patient or the animal, the route of administration, the dosage form and the like. In general, the suitable daily dosage of the phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the present application ranges from 0.001 mg/kg of body weight to 150 mg/kg of body weight, preferably, from 0.1 mg/kg of body weight to 100 mg/kg of body weight, more preferably, from 1 mg/kg of body weight to 70 mg/kg of body weight, and most preferably, from 2 mg/kg of body weight to 30 mg/kg of body weight. The above dosage may be administered as a single dosage unit or divided into several dosage units, depending on the physician's clinical experience and the dosage regimen of other therapeutic means used together.

The phenyl acrylic acid compound described in the first aspect, the tautomer, enantiomer or diastereomer of the phenyl acrylic acid compound or the pharmaceutically acceptable salt thereof described in the second aspect, or the pharmaceutical composition described in the fourth aspect in the present application may be used alone or in combination with other therapeutic drugs or drugs required for the treatment for symptoms. When synergistic effects exist with other therapeutic drugs, the dosage should be adjusted according to the actual situation.

Compared with the prior art, the present application has the beneficial effects described below.

The phenyl acrylic compound provided by the present application can be well combined with a plurality of target proteins, has good bioavailability, and can be used for preparing drugs for preventing or treating tumors, autoimmune diseases, inflammatory diseases, neurodegenerative diseases or aging, where the tumor is glioma, melanoma, gastric cancer, lung cancer, breast cancer, kidney cancer, liver cancer, oral epithelial carcinoma, head and neck tumor, cervical cancer, ovarian cancer, pancreatic cancer, prostate cancer, colon cancer, rectal adenocarcinoma, leukemia or lymphoma.

### DETAILED DESCRIPTION

Technical solutions of the present application will be further described below through specific examples. Those skilled in the art are to understand that the examples set forth below are used for a better understanding of the present application and are not to be construed as specific limitations on the present application.

The reaction raw materials in the following examples are commercially available conventional products.

### Preparation example 1

This preparation example provides Compound c-1, and the method for preparing Compound c-1 includes the following steps. 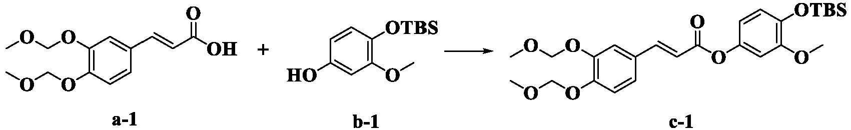 (E)-3-(3,4-bis(methoxymethoxy)phenyl)acrylic acid (a-1) (536 mg, 2 mmol), 4-tert-butyldimethylsilyloxy-3-methoxyphenol (b-1) (559 mg, 2.2 mmol), EDCI (575 mg, 3 mmol) and 10 mg of DMAP were dissolved in 20 mL of dichloromethane, triethylamine (0.56 mL, 4 mmol) was dropped at 0 °C, and the reaction was carried out at 25 °C for 10 hours. After the reaction was completed, ethyl acetate and water were added, and liquid separation was carried out to collect the organic phase. The organic phase was dried with anhydrous sodium sulfate, filtered, concentrated, and separated by column chromatography (PE:EA = 5:1) to give 0.63 g of Compound c-1 as yellow solidified oil, with a yield of 62.4%.

¹H NMR (500 MHz, CDCl₃) *δ* 7.79 (s, 1H), 7.42 (s, 1H), 7.19 (s, 2H), 6.83 (d, *J* = 8.5 Hz, 1H), 6.69 (s, 1H), 6.63 (dd, *J* = 8.6, 2.7 Hz, 1H), 6.49 (d, *J* = 15.9 Hz, 1H), 5.27 (d, *J* = 5.3 Hz, 4H), 3.79 (s, 3H), 3.54 (s, 3H), 3.52 (s, 3H), 1.00 (s, 9H), 0.16 (s, 6H).

¹³C NMR (101 MHz, CDCl₃) *δ* 165.8, 151.2, 149.5, 147.5, 146.0, 145.1, 142.7, 128.7, 123.8, 120.6, 116.2, 115.9, 115.8, 113.6, 106.2, 95.6, 94.8, 56.4, 56.3, 55.6, 25.7, 18.5, -4.6.

### Preparation example 2

This preparation example provides Compound c-2. With Compound a-1 (536 mg, 2 mmol) and Compound b-2 (557 mg, 2.2 mmol) as raw materials, Compound c-2 was prepared in the same method as Compound c-1, and 600 mg of Compound c-2 was given as a white solid, with a yield of 62%. The reaction formula of Compound c-2 is shown below:

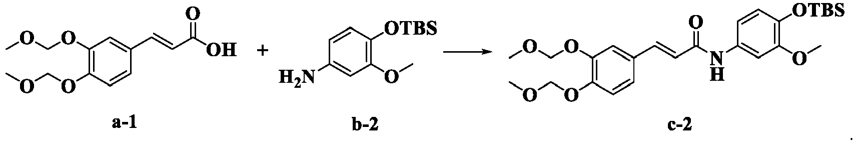

¹H NMR (400 MHz, CDCl₃) *δ* 7.75 (d, *J* = 15.2 Hz, 1H), 7.65-7.67 (m, 2H), 7.47 (m, 1H), 7.42 (d, *J =* 15.2 Hz, 1H), 7.26-7.28 (m, 1H), 7.19 (d, *J =* 8.0 Hz, 1H), 7.05 (d, *J =* 8 Hz, 1H), 5.22 (s, 2H), 5.22 (s, 2H), 3.90 (s, 3H), 3.60 (s, 3H), 3.53 (s, 3H), 1.03 (s, 9H), 0.33 (s, 6H).

### Preparation example 3

This preparation example provides Compound c-3. With Compound a-1 (536 mg, 2 mmol) and Compound b-3 (490 mg, 2.2 mmol) as raw materials, Compound c-3 was prepared in the same method as Compound c-1, and 650 mg of Compound c-3 was given as a white solid, with a yield of 68.4%. The reaction formula of Compound c-3 is shown below:

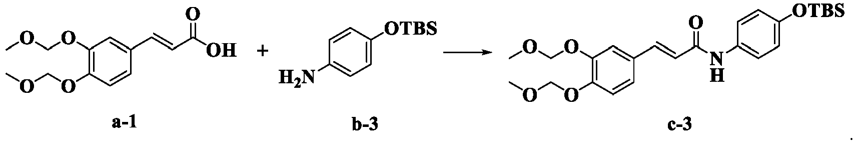

¹H NMR (400 MHz, CDCl₃) *δ* 7.80 (d, *J* = 15.6 Hz, 1H), 7.63-7.68 (m, 2H), 7.49 (m, 1H), 7.41 (d, *J =* 15.6 Hz, 1H), 7.26-7.28 (m, 1H), 7.19 (d, *J =* 8.8 Hz, 1H), 7.05 (d, *J =* 8.8 Hz, 1H), 5.24 (s, 2H), 5.24 (s, 2H), 3.60 (s, 3H), 3.53 (s, 3H), 1.02 (s, 9H), 0.34 (s, 6H).

### Preparation example 4

This preparation example provides Compound d-1, and the method for preparing Compound d-1 includes the following steps.

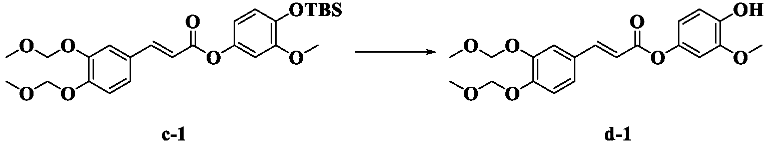

Compound c-1 (500 mg, 1 mmol) was dissolved in THF, tetrabutylammonium fluoride (1.5 mL, 1.5 mmol) was added at 0 °C, and 30 minutes later, saturated ammonium chloride was added to terminate the reaction. Ethyl acetate was added, and liquid separation was carried out. The organic phase was dried with anhydrous sodium sulfate, filtered, concentrated, and separated by column chromatography (PE:EA = 5:1) to give 0.35 g of Compound d-1 as a yellow solid, with a yield of 89.7%.

¹H NMR (400 MHz, CDCl₃) *δ* 7.77 (d, *J* = 15.9 Hz, 1H), 7.42 (s, 1H), 7.18 (s, 2H), 6.90 (d, *J* = 8.5 Hz, 1H), 6.70 (d, *J* = 2.6 Hz, 1H), 6.65 (dd, *J* = 8.5, 2.6 Hz, 1H), 6.49 (d, *J* = 15.9 Hz, 1H), 5.68 (s, 1H), 5.27 (d, *J =* 4.2 Hz, 4H), 3.85 (s, 3H), 3.53 (s, 3H), 3.52 (s, 3H).

¹³C NMR (101 MHz, CDCl₃) *δ* 166.1, 149.6, 147.5, 146.7, 146.1, 143.8, 143.4, 128.7, 123.8, 116.2, 115.8, 115.8, 114.4, 113.8, 105.3, 95.6, 95.1, 77.4, 77.1, 76.8, 56.4, 56.3, 56.0.

### Preparation example 5

This preparation example provides Compound d-2. With Compound c-2 (500 mg, 1 mmol) as the raw material, Compound d-2 was prepared in the same method as Compound d-1, and 352 mg of Compound d-2 was given as a pale yellow solid, with a yield of 90.1%. The reaction formula of Compound d-2 is shown below:

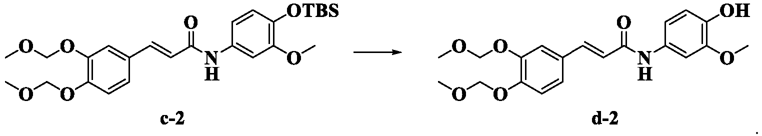

¹H NMR (400 MHz, CDCl₃) *δ* 9.90 (s, 1H), 9.17 (s, 1H), 7.48 (d, J = 8.9 Hz, 2H), 7.45 (d, J = 15.7 Hz, 1H), 7.38 (d, J = 2.0 Hz, 1H), 7.25 - 7.09 (m, 1H), 6.73 (d, J = 8.9 Hz, 1H), 6.65 (d, J = 15.6 Hz, 1H), 5.29 (s, 2H), 5.29 (s, 2H), 4.12 (s, 3H), 3.56 (s, 3H), 3.53 (s, 3H).

### Preparation example 6

This preparation example provides Compound d-3. With Compound c-3 (470 mg, 1 mmol) as the raw material, Compound d-3 was prepared in the same method as Compound d-1, and 340 mg of Compound d-3 was given as a pale yellow solid, with a yield of 94.7%. The reaction formula of Compound d-3 is shown below:

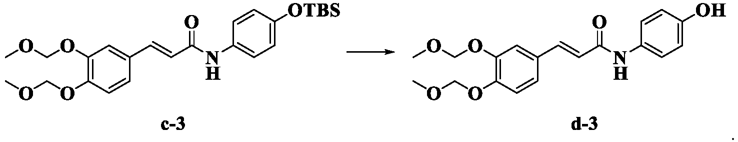

¹H NMR (400 MHz, *d*-DMSO) *δ* 9.91 (s, 1H), 9.16 (s, 1H), 7.47 (d, J = 8.9 Hz, 2H), 7.43 (d, J = 15.7 Hz, 1H), 7.36 (d, J = 2.0 Hz, 1H), 7.21 - 7.09 (m, 1H), 6.70 (d, J = 8.9 Hz, 1H), 6.64 (d, J = 15.6 Hz, 1H), 5.24 (s, 2H), 5.22 (s, 2H), 3.42 (s, 3H), 3.40 (s, 3H).

¹³C NMR (101 MHz, d-DMSO) *δ* 163.0, 153.3, 148.2, 147.0, 139.0, 131.1, 129.1, 122.9, 120.9, 120.7, 116.8, 115.1, 115.1, 94.8, 94.6, 55.8, 40.2, 39.9, 39.7, 39.5, 39.3, 39.1, 38.9.

### Example 1

This example provides Compound M1, and the method for preparing Compound M1 includes the following steps:

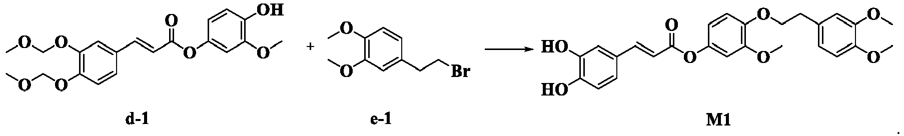

Compound d-1 (50 mg, 0.13 mmol), 3,4-dimethoxyphenethyl bromide (e-1) (50 mg, 0.2 mmol), cesium carbonate (85 mg, 0.26 mmol) and 5 mL of acetonitrile were loaded into a 50 mL roundbottomed flask, and the reaction was carried out at reflux for 4 hours until TLC showed that the reaction was completed. After the reaction was cooled, 60 mg of the crude product was obtained by filtration. The crude product was dissolved in 5 mL of tetrahydrofuran, 0.3 mL of concentrated hydrochloric acid was added, and the mixture was stirred for 1 hour and concentrated to give 47 mg of compound M1, with a yield of 78.3%.

¹H NMR (400 MHz, CDCl₃) *δ* 7.71 (d, *J* = 15.9 Hz, 1H), 7.09 (d, *J* = 2.0 Hz, 1H), 7.03 (dd, *J* = 8.2, 2.0 Hz, 1H), 6.89 (d, *J =* 2.1 Hz, 1H), 6.85 (d, *J =* 7.9 Hz, 2H), 6.80 (d, *J =* 1.1 Hz, 2H), 6.73 - 6.67 (m, 2H), 6.36 (d, *J* = 15.9 Hz, 1H), 5.97 - 5.86 (m, 2H), 4.16 (t, *J* = 7.3 Hz, 2H), 3.88 - 3.84 (m, 9H), 3.09 (t, *J* = 7.3 Hz, 2H).

¹³C NMR (151 MHz, CDCl₃) *δ* 166.3, 149.3, 148.6, 147.6, 147.0, 146.7, 146.6, 144.9, 143.9, 131.4, 127.3, 123.6, 121.0, 116.5, 114.6, 114.3, 113.2, 112.5, 111.9, 111.3, 107.4, 70.0, 58.0, 55.9, 55.3, 35.2.

### Example 2

This example provides Compound M2. With Compound d-1 (50 mg, 0.13 mmol) and Compound e-2 (80 mg, 0.22 mmol) as raw materials, Compound M2 was prepared in the same method as Compound M1, and 54 mg of Compound M2 was given as a yellow solid, with a yield of 84.2%. The reaction formula of Compound M2 is shown below:

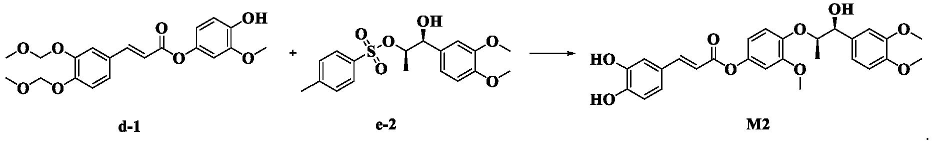

¹H NMR (400 MHz, CDCl₃) *δ* 7.73 (d, *J* = 15.9 Hz, 1H), 7.11 (d, *J* = 2.0 Hz, 1H), 7.06 (dd, *J* = 8.2, 2.1 Hz, 1H), 7.01 (d*, J =* 1.8 Hz, 1H), 6.93 (d, *J =* 8.9 Hz, 1H), 6.90 - 6.85 (m, 3H), 6.83 (dd, *J =* 8.6, 2.7 Hz, 2H), 6.39 (d, *J =* 15.8 Hz, 1H), 4.89 (d, *J =* 3.2 Hz, 1H), 4.43 (qd, *J =* 6.3, 3.1 Hz, 1H), 3.91 - 3.83 (m, 9H), 1.20 (d, *J =* 6.4 Hz, 3H).

¹³C NMR (151 MHz, CDCl₃) *δ* 166.2, 149.4, 148.8, 148.3, 146.9, 146.0, 144.9, 143.9, 132.2, 127.2, 122.8, 118.7, 115.6, 114.5, 113.1, 112.6, 110.8, 109.6, 82.0, 74.6, 58.3, 53.9, 28.7, 12.4.

### Example 3

This example provides Compound M3. With Compound d-1 (50 mg, 0.14 mmol) and Compound e-3 (80 mg, 0.22 mmol) as raw materials, Compound M3 was prepared in the same method as Compound M1, and 45 mg of Compound M3 was given as a yellow solid, with a yield of 64.3%.

The reaction formula of Compound M3 is shown below:

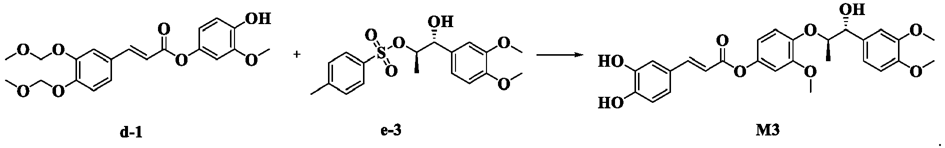

¹H NMR (400 MHz, CDCl₃) *δ* 7.73 (d, J = 15.9 Hz, 1H), 7.11 (d, J = 2.0 Hz, 1H), 7.04 (dd, J = 8.2, 2.1 Hz, 1H), 6.95 - 6.89 (m, 3H), 6.89 - 6.78 (m, 4H), 6.38 (d, J = 16.0 Hz, 1H), 4.68 (d, J = 8.4 Hz, 1H), 4.26 - 4.18 (m, 1H), 3.90 (s, 3H), 3.87 (s, 3H), 3.85 (s, 3H), 1.18 (d, J = 6.3 Hz, 3H).

### Example 4

This example provides Compound M4. With Compound d-3 (130 mg, 0.36 mmol) and Compound e-4 (100 mg, 0.54 mmol) as raw materials, Compound M4 was prepared in the same method as Compound M1, and 100 mg of Compound M4 was given as a yellow solid, with a yield of 88.5%. The reaction formula of Compound M4 is shown below:

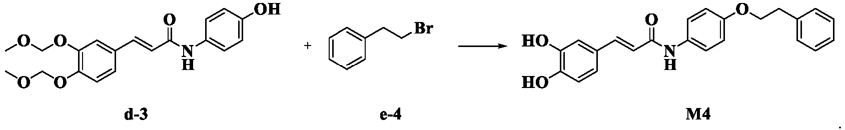

¹H NMR (400 MHz, Acetone-*d*₆) *δ* 7.63 (d, *J* = 9.1 Hz, 2H), 7.48 (d, *J =* 15.5 Hz, 1H), 7.37 - 7.25 (m, 4H), 7.19 (t, *J* = 6.3 Hz, 1H), 7.07 (d, *J* = 2.1 Hz, 1H), 6.95 (dd, *J* = 8.2, 2.1 Hz, 1H), 6.89 - 6.76 (m, 3H), 6.54 (d, *J =* 15.5 Hz, 1H), 4.16 (t, *J =* 6.9 Hz, 2H), 3.04 (t, *J =* 6.9 Hz, 3H).

¹³C NMR (101 MHz, Acetone-*d*₆) *δ* 164.3, 156.7, 149.0, 146.0, 140.6, 138.9, 133.8, 130.1, 129.0, 128.3, 127.0, 122.5, 121.3, 119.7, 116.2, 115.3, 113.3, 68.5, 36.3.

### Example 5

This example provides Compound M5. With Compound d-3 (150 mg, 0.42 mmol) and Compound e-5 (134 mg, 0.63 mmol) as raw materials, Compound M5 was prepared in the same method as Compound M1, and 150 mg of Compound M5 was given as a yellow solid, with a yield of 88.2%.

The reaction formula of Compound M5 is shown below:

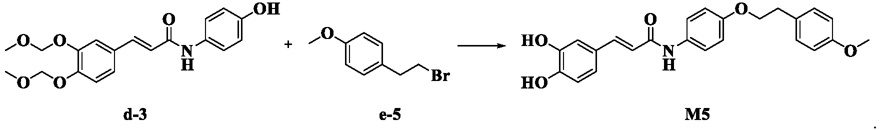

¹H NMR (400 MHz, Acetone-*d*₆) *δ* 8.05 (d, *J =* 3.6 Hz, 1H), 7.61 (s, 2H), 7.47 (d, *J =* 15.5 Hz, 1H), 7.22 (d, *J =* 8.5 Hz, 2H), 7.06 (s, 1H), 6.94 (d, *J =* 8.1 Hz, 1H), 6.87 - 6.77 (m, 5H), 6.53 (d, *J =* 15.5 Hz, 1H), 4.10 (t, *J* = 6.9 Hz, 2H), 2.96 (t, *J* = 6.9 Hz, 2H).

¹³C NMR (101 MHz, Acetone-*d*₆) *δ* 163.9, 158.4, 155.0, 147.0, 145.4, 140.6, 130.5, 129.9, 127.5, 120.9, 120.6, 120.5, 119.0, 115.5, 115.4, 114.5, 114.0, 113.9, 113.7, 68.9, 55.0, 36.2.

### Example 6

This example provides Compound M6. With Compound d-3 (150 mg, 0.42 mmol) and Compound e-6 (126 mg, 0.63 mmol) as raw materials, Compound M6 was prepared in the same method as Compound M1, and 100 mg of Compound M6 was given as a yellow solid, with a yield of 60.6%. The reaction formula of Compound M6 is shown below:

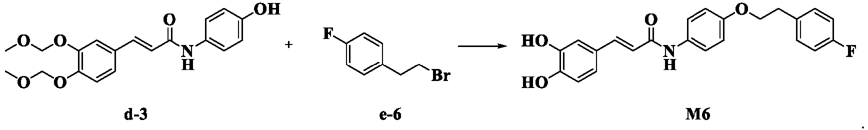

¹H NMR (400 MHz, Acetone-*d*₆) *δ* 9.07 (s, 1H), 8.30 (s, 1H), 8.06 (s, 1H), 7.63 (d, *J* = 9.0 Hz, 2H), 7.46 (s, 1H), 7.35 (dd, *J =* 8.5, 5.7 Hz, 2H), 7.10 - 7.00 (m, 3H), 6.94 (dd, *J =* 8.2, 2.1 Hz, 1H), 6.86 - 6.77 (m, 2H), 6.54 (d, *J =* 15.6 Hz, 1H), 4.15 (t, *J =* 6.8 Hz, 2H), 3.04 (d, *J =* 6.8 Hz, 2H).

¹³C NMR (101 MHz, Acetone) *δ* 165.0, 162.8, 160.9, 155.4, 147.9, 145.4, 139.6, 134.9, 134.8, 133.1, 130.8, 130.7, 127.5, 120.9, 120.6, 120.5, 119.0, 115.5, 115.0, 114.7, 114.5, 114.0, 66.1, 34.6.

### Example 7

This example provides Compound M7. With Compound d-3 (150 mg, 0.42 mmol) and Compound e-7 (125 mg, 0.63 mmol) as raw materials, Compound M7 was prepared in the same method as Compound M1, and 101 mg of Compound M7 was given as a yellow solid, with a yield of 61.9%. The reaction formula of Compound M7 is shown below:

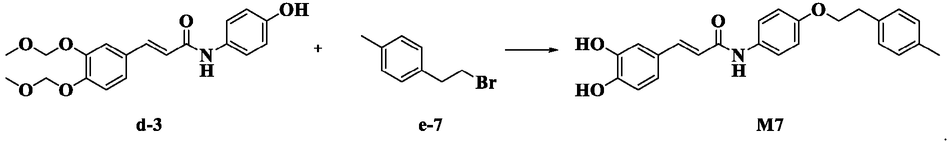

¹H NMR (400 MHz, Acetone-*d*₆) *δ* 9.06 (s, 1H), 8.30 (s, 1H), 8.06 (d, *J* = 3.5 Hz, 1H), 7.63 (d, *J* = 8.5 Hz, 2H), 7.48 (d, *J =* 15.5 Hz, 1H), 7.19 (d, *J =* 8.0 Hz, 2H), 7.12 - 7.03 (m, 3H), 6.94 (dd, *J =* 8.1, 2.1 Hz, 1H), 6.84 (dd, *J* = 14.3, 8.6 Hz, 3H), 6.54 (d, *J* = 15.5 Hz, 1H), 4.12 (t, *J* = 6.9 Hz, 2H), 2.99 (t, *J* = 7.0 Hz, 2H).

¹³C NMR (101 MHz, Acetone) *δ* 164.1, 153.4, 147.1, 145.9, 140.6, 136.4, 133.7, 128.9, 127.5, 120.9, 120.6, 119.0, 115.5, 114.5, 111.9, 70.5, 68.8, 38.0, 21.4.

### Example 8

This example provides Compound M8. With Compound d-3 (150 mg, 0.42 mmol) and Compound e-8 (126 mg, 0.63 mmol) as raw materials, Compound M8 was prepared in the same method as Compound M1, and 80 mg of Compound M8 was given as a yellow solid, with a yield of 50%. The reaction formula of Compound M8 is shown below:

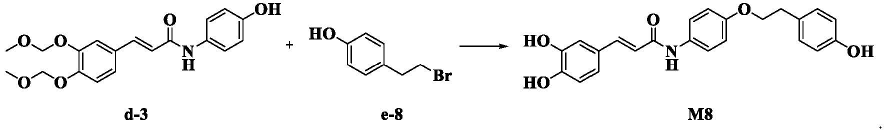

¹H NMR (400 MHz, Acetone-*d*₆) *δ* 9.07 (s, 1H), 8.33 (s, 1H), 8.09 (s, 1H), 7.63 (d, *J* = 9.0 Hz, 2H), 7.48 (d, *J =* 15.5 Hz, 1H), 7.13 (d, *J =* 8.4 Hz, 2H), 7.07 (d, *J =* 2.1 Hz, 1H), 6.94 (dd, *J =* 8.2, 2.1 Hz, 1H), 6.84 (dd, *J* = 14.4, 8.6 Hz, 3H), 6.75 (d, *J* = 8.5 Hz, 2H), 6.49 (s, 1H), 4.09 (t, *J* = 7.0 Hz, 2H), 2.94 (t, *J* = 7.0 Hz, 2H).

¹³C NMR (101 MHz, Acetone) *δ* 161.4, 156.0, 153.7, 148.1, 143.8, 140.6, 130.5, 128.5, 125.5, 121.6, 120.6, 118.3, 115.5, 115.1, 114.5, 114.0, 69.1, 35.3.

### Example 9

This example provides Compound M9. With Compound d-3 (150 mg, 0.42 mmol) and Compound e-1 (153 mg, 0.63 mmol) as raw materials, Compound M9 was prepared in the same method as Compound M1, and 100 mg of Compound M9 was given as a pale yellow solid, with a yield of 54.6%. The reaction formula of Compound M9 is shown below:

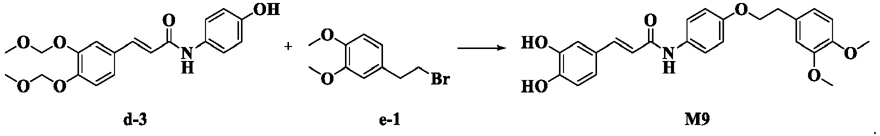

¹H NMR (400 MHz, Acetone-d₆) *δ* 9.05 (s, 1H), 8.30 (s, 1H), 8.05 (s, 1H), 7.63 (d, *J* = 8.6 Hz, 2H), 7.48 (d, *J =* 15.5 Hz, 1H), 7.06 (d, *J* = 2.1 Hz, 1H), 6.95 (d, *J =* 9.0 Hz, 2H), 6.89 - 6.78 (m, 4H), 6.54 (d, *J =* 15.5 Hz, 1H), 4.13 (t, *J =* 7.0 Hz, 2H), 2.97 (t, *J=* 7.0 Hz, 2H).

¹³C NMR (101 MHz, Acetone) *δ* 164.0, 155.0, 150.6, 148.6, 147.1, 144.7, 141.3, 135.2, 131.1, 128.1, 121.0, 120.6, 119.0, 116.1, 114.5, 114.0, 113.2, 112.1, 68.9, 55.3, 55.2, 37.4.

### Example 10

This example provides Compound M10. With Compound d-3 (150 mg, 0.42 mmol) and Compound e-9 (173 mg, 0.63 mmol) as raw materials, Compound M10 was prepared in the same method as Compound M1, and 95 mg of Compound M10 was given as a pale yellow solid, with a yield of 48.7%. The reaction formula of Compound M10 is shown below:

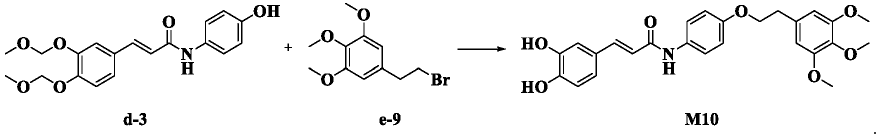

¹H NMR (400 MHz, Acetone-*d*₆) *δ* 9.15 (s, 1H), 8.32 (s, 1H), 8.08 (s, 1H), 7.61 (s, 2H), 7.46 (d, *J = 15.6* Hz, 1H), 7.04 (s, 1H), 6.92 (d, *J* = 6.3 Hz, 1H), 6.83 (d, *J* = 17.2 Hz, 3H), 6.61 (s, 2H), 6.53 (d, *J =* 15.2 Hz, 1H), 4.13 (s, 2H), 3.76 (s, 7H), 3.65 (s, 3H), 2.95 (s, 2H).

¹³C NMR (101 MHz, Acetone) *δ* 163.7, 155.0, 152.4, 147.2, 145.4, 141.3, 136.9, 134.2, 133.1, 127.9, 121.3, 120.7, 118.4, 115.5, 114.6, 113.7, 107.0, 68.4, 59.0, 56.3, 37.3.

### Example 11

This example provides Compound M11. With Compound d-3 (130 mg, 0.36 mmol) and Compound e-4 (100 mg, 0.54 mmol) as raw materials, Compound M11 was prepared in the same method as Compound M1, without the step of acidification, and 110 mg of Compound M11 was given as a pale yellow solid, with a yield of 66.3%. The reaction formula of Compound M11 is shown below:

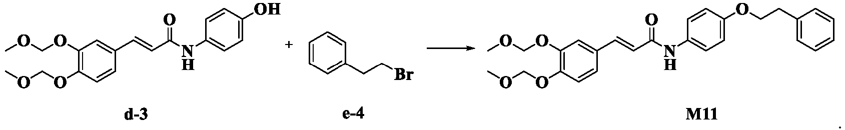

¹H NMR (400 MHz, Chloroform-d) *δ* 7.63 (d, *J* = 15.5 Hz, 1H), 7.49 (d, *J* = 8.3 Hz, 2H), 7.38 - 7.20 (m, 8H), 7.12 (d, *J =* 5.8 Hz, 2H), 6.86 (d, *J =* 9.0 Hz, 2H), 6.38 (d, *J =* 15.4 Hz, 1H), 5.25 (s, 4H), 4.15 (t, *J* = 7.1 Hz, 2H), 3.52 (s, 2H), 3.50 (s, 3H), 3.08 (t, *J* = 7.1 Hz, 2H).

¹³C NMR (101 MHz, CDCl₃) *δ* 162.9, 157.4, 148.8, 146.2, 142.1, 137.7, 132.3, 129.7, 129.0, 128.5, 126.5, 123.5, 121.6, 119.9, 116.2, 115.0, 95.9, 94.4, 70.9, 56.4, 34.8.

### Example 12

This example provides Compound M12. With Compound d-3 (150 mg, 0.42 mmol) and Compound e-5 (134 mg, 0.63 mmol) as raw materials, Compound M12 was prepared in the same method as Compound M1, without the step of acidification, and 180 mg of Compound M11 was given as a pale yellow solid, with a yield of 87%. The reaction formula of Compound M12 is shown below:

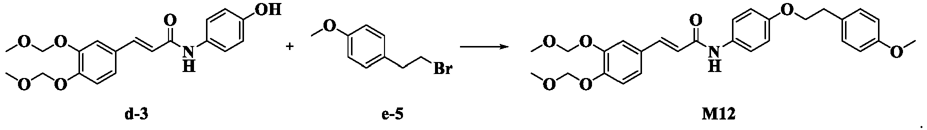

¹H NMR (400 MHz, Chloroform-d) *δ* 7.64 (d, *J* = 15.4 Hz, 1H), 7.49 (d, *J* = 10.3 Hz, 2H), 7.37 (s, 1H), 7.19 (d, *J* = 8.6 Hz, 3H), 7.13 (d, *J =* 3.9 Hz, 2H), 6.85 (dd, *J =* 8.7, 6.2 Hz, 4H), 6.38 (d, *J =* 15.5 Hz, 1H), 5.25 (s, 5H), 4.10 (d, *J =* 7.1 Hz, 2H), 3.02 (d, *J =* 7.0 Hz, 2H).

¹³C NMR (101 MHz, CDCl₃) *δ* 163.9, 158.3, 155.7, 149.6, 148.1, 140.5, 131.3, 130.7, 130.0, 127.7, 122.9, 121.6, 120.3, 117.2, 114.6, 114.0, 100.1, 94.7, 68.1, 57.2, 55.3, 34.9.

### Example 13

This example provides Compound M13. With Compound d-3 (150 mg, 0.42 mmol) and Compound e-6 (125 mg, 0.62 mmol) as raw materials, Compound M13 was prepared in the same method as Compound M1, without the step of acidification, and 120 mg of Compound M13 was given as a pale yellow solid, with a yield of 59.4%. The reaction formula of Compound M13 is shown below:

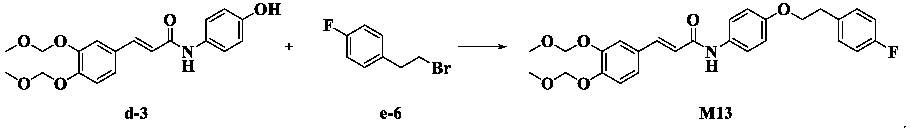

¹H NMR (400 MHz, CDCl₃) *δ* 7.67 (s, 1H), 7.51 (d, *J =* 8.2 Hz, 2H), 7.38 (s, 1H), 7.28 - 7.18 (m, 4H), 7.14 (d, *J* = 4.0 Hz, 2H), 7.00 (t, *J* = 8.7 Hz, 2H), 6.87 (d, *J* = 9.0 Hz, 2H), 6.40 (d, *J* = 15.4 Hz, 1H), 5.27 (s, 4H), 4.14 (t, *J* = 6.9 Hz, 2H), 3.53 (d, *J* = 9.6 Hz, 6H).

¹³C NMR (101 MHz, CDCl₃) *δ* 163.8, 162.9, 159.6, 154.0, 148.8, 146.7, 141.5, 134.7, 131.4, 130.4, 130.3, 128.3, 123.5, 121.5, 119.4, 116.2, 115.3, 115.1, 114.9, 68.4, 53.6, 36.0.

### Example 14

This example provides Compound M14. With Compound d-3 (150 mg, 0.42 mmol) and Compound e-7 (125 mg, 0.62 mmol) as raw materials, Compound M14 was prepared in the same method as Compound M1, without the step of acidification, and 150 mg of Compound M14 was given as a pale yellow solid, with a yield of 75%. The reaction formula of Compound M14 is shown below:

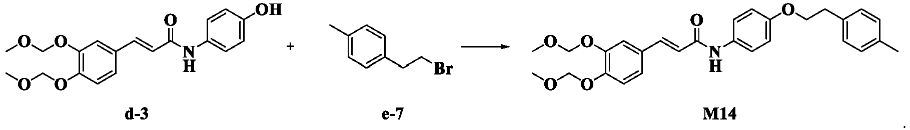

¹H NMR (400 MHz, d-DMSO) *δ* 10.55 (s, 1H), 8.68 (d, *J =* 1.6 Hz, 1H), 7.80-7.82 (m, 2H), 7.58 (dd, *J* = 3.0 Hz, 8.8 Hz, 1H), 7.21-7.23 (m, 2H), 7.09-7.12 (m, 3H), 6.96-7.01 (m, 1H), 6.77-6.80 (m, 1H), 4.23 (t, *J =* 7.2 Hz, 2H), 3.82 (s, 3H), 3.02 (t, *J =* 7.2 Hz, 2H) , 2.26 (s, 3H).

¹³C NMR (101 MHz, CDCl₃) *δ* 165.4, 154.8, 148.8, 147.1, 142.0, 136.1, 133.6, 129.8, 128.4, 123.5, 121.5, 119.5, 116.2, 112.0, 95.5, 94.8, 68.3, 56.4, 35.4, 20.5.

### Example 15

This example provides Compound M15. With Compound d-3 (60 mg, 0.22 mmol) and Compound e-8 (25 mg, 0.18 mmol) as raw materials, Compound M15 was prepared in the same method as Compound M1, without the step of acidification, and 60 mg of Compound M15 was given as a pale yellow solid, with a yield of 83.4%. The reaction formula of Compound M15 is shown below:

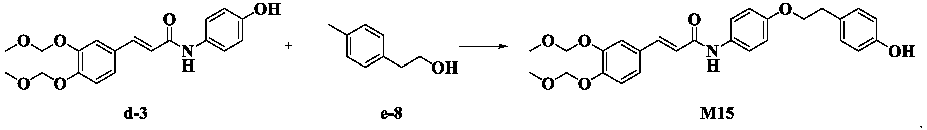

¹H NMR (400 MHz, Chloroform-d) *δ* 7.65 (d, *J* = 15.4 Hz, 1H), 7.54 - 7.33 (m, 3H), 7.15 (dd, *J* = 13.9, 6.6 Hz, 5H), 6.92 - 6.73 (m, 5H), 6.40 (d, *J* = 13.7 Hz, 1H), 5.26 (s, 5H), 4.11 (t, *J* = 6.3 Hz, 3H), 3.54 (s, 3H), 3.52 (s, 3H), 3.01 (t, *J* = 7.0 Hz, 3H).

¹³C NMR (101 MHz, CDCl₃) *δ* 164.0, 156.7, 155.8, 154.8, 149.4, 147.4, 141.1, 131.2, 130.1, 130.0, 129.2, 123.6, 122.1, 119.4, 117.0, 115.4, 115.0, 114.7, 95.5, 94.8, 77.3, 69.3, 68.4, 56.3, 34.9.

### Example 16

This example provides Compound M16. With Compound d-3 (150 mg, 0.42 mmol) and Compound e-1 (206 mg, 0.84 mmol) as raw materials, Compound M16 was prepared in the same method as Compound M1, without the step of acidification, and 150 mg of Compound M16 was given as a pale yellow solid, with a yield of 68.2%. The reaction formula of Compound M16 is shown below:

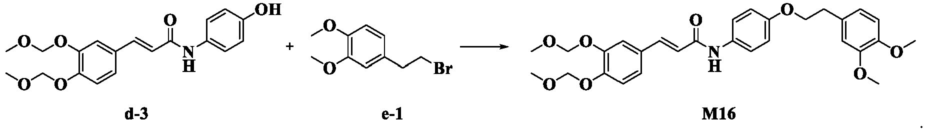

¹H NMR (400 MHz, Chloroform-*d*) *δ* 7.65 (d, *J =* 15.3 Hz, 1H), 7.51 (d, *J* = 8.2 Hz, 2H), 7.35 (d, *J = 20.2* Hz, 2H), 7.13 (q, *J =* 8.1 Hz, 2H), 6.88 (d, *J =* 9.0 Hz, 2H), 6.82 (s, 3H), 6.40 (d, *J =* 15.4 Hz, 1H), 4.14 (t, *J =* 7.0 Hz, 2H), 3.88 (s, 3H), 3.87 (s, 3H), 3.54 (s, 3H), 3.51 (s, 3H), 3.03 (t, *J* = 7.0 Hz, 2H).

¹³C NMR (101 MHz, CDCl₃) *δ* 163.5, 156.1, 149.6, 147.7, 147.4, 138.2, 130.8, 128.4, 123.5, 122.5, 120.9, 119.0, 117.3, 113.9, 112.4, 110.8, 96.0, 93.9, 68.1, 57.7, 55.9, 36.1, 29.7.

### Example 17

This example provides Compound M17. With Compound d-3 (150 mg, 0.42 mmol) and Compound e-9 (173 mg, 0.63 mmol) as raw materials, Compound M17 was prepared in the same method as Compound M1, without the step of acidification, and 106 mg of Compound M17 was given as a pale yellow solid, with a yield of 45.7%. The reaction formula of Compound M17 is shown below:

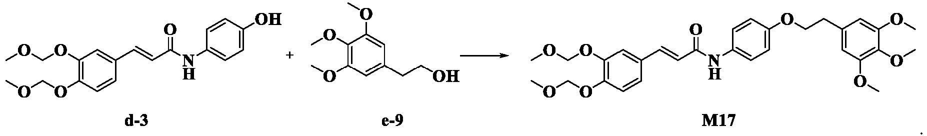

¹H NMR (400 MHz, Chloroform-*d*) *δ* 7.66 (d, *J =* 15.4 Hz, 1H), 7.51 (d, *J =* 7.9 Hz, 2H), 7.39 (s, 1H), 7.23 - 7.11 (m, 3H), 6.89 (d, *J* = 8.8 Hz, 2H), 6.50 (s, 2H), 6.40 (d, *J* = 15.4 Hz, 1H), 5.27 (s, 4H), 4.17 (t, *J =* 7.0 Hz, 2H), 3.86 (s, 6H), 3.84 (s, 3H), 3.54 (s, 3H), 3.52 (s, 3H), 3.03 (t, *J =* 7.0 Hz, 2H).

¹³C NMR (101 MHz, CDCl₃) *δ* 152.0, 148.9, 147.5, 141.6, 135.4, 132.8, 127.2, 123.1, 120.6, 119.4, 117.3, 115.0, 113.5, 106.0, 95.9, 95.2, 77.3, 69.1, 68.6, 62.2, 59.4, 56.4, 54.8.

### Example 18

This example provides Compound M18. With Compound d-1 (50 mg, 0.13 mmol) and Compound e-4 (37 mg, 0.2 mmol) as raw materials, Compound M18 was prepared in the same method as Compound M1, and 30 mg of Compound M18 was given as a pale yellow solid, with a yield of 56.6%. The reaction formula of Compound M18 is shown below:

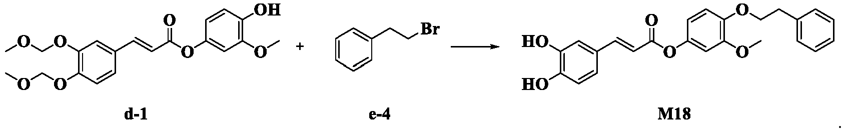

¹H NMR (400 MHz, Acetone-*d*₆) *δ* 7.60 (d, *J =* 9.1 Hz, 2H), 7.45 (d, *J =* 15.5 Hz, 1H), 7.34 - 7.25 (m, 4H), 7.16 (t, *J* = 6.3 Hz, 1H), 7.04 (d, *J* = 2.1 Hz, 1H), 6.95 (dd, *J* = 8.2, 2.1 Hz, 1H), 6.89 - 6.76 (m, 3H), 6.54 (d, *J =* 15.5 Hz, 1H), 4.13 (t, *J =* 6.9 Hz, 2H), 3.84 (s, 3H), 3.01 (t, *J =* 6.9 Hz, 3H).

### Example 19

This example provides Compound M19. With Compound d-1 (100 mg, 0.26 mmol) and Compound e-6 (66 mg, 0.33 mmol) as raw materials, Compound M19 was prepared in the same method as Compound M1, and 60 mg of Compound M19 was given as a pale yellow solid, with a yield of 54.4%. The reaction formula of Compound M19 is shown below:

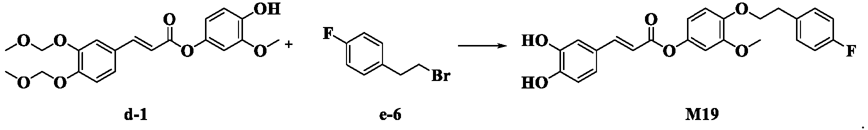

¹H NMR (400 MHz, Acetone-*d*₆) *δ* 9.05 (s, 1H), 8.32 (s, 1H), 8.04 (s, 1H), 7.63 (d, *J* = 9.0 Hz, 2H), 7.46 (s, 1H), 7.35 (dd, *J* = 8.5, 5.7 Hz, 2H), 7.10 - 7.00 (m, 3H), 6.94 (dd, *J* = 8.2, 2.1 Hz, 1H), 6.86 - 6.77 (m, 2H), 6.54 (d, *J =* 15.6 Hz, 1H), 4.15 (t, *J =* 7.2 Hz, 2H), 3.84 (s, 3H), 3.02 (d, *J = 6.8* Hz, 2H).

### Example 20

This example provides Compound M20. With Compound d-1 (50 mg, 0.13 mmol) and Compound e-5 (70 mg, 0.33 mmol) as raw materials, Compound M20 was prepared in the same method as Compound M1, and 40 mg of Compound M20 was given as a pale yellow solid, with a yield of 70.2%. The reaction formula of Compound M20 is shown below:

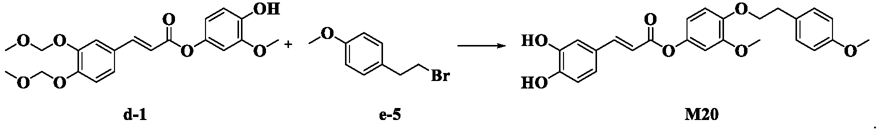

¹H NMR (400 MHz, Acetone-*d*₆) *δ* 8.07 (d, *J* = 3.6 Hz, 1H), 7.63 (s, 2H), 7.47 (d, *J* = 15.6 Hz, 1H), 7.22 (d, *J* = 8.6 Hz, 2H), 7.06 (s, 1H), 6.94 (d, *J =* 8.1 Hz, 1H), 6.87 - 6.77 (m, 5H), 6.53 (d, *J =* 15.5 Hz, 1H), 4.12 (t, *J =* 6.9 Hz, 2H), 3.84 (s, 3H), 2.96 (t, *J* = 6.9 Hz, 2H).

### Example 21

This example provides Compound M21. With Compound d-1 (50 mg, 0.13 mmol) and Compound e-7 (45 mg, 0.23 mmol) as raw materials, Compound M21 was prepared in the same method as Compound M1, and 40 mg of Compound M21 was given as a pale yellow solid, with a yield of 72.7%. The reaction formula of Compound M21 is shown below:

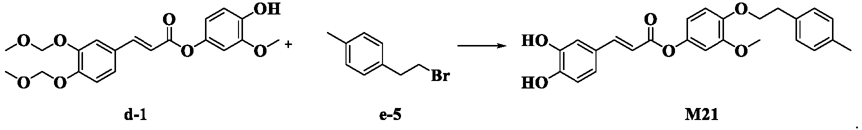

¹H NMR (400 MHz, *d*-DMSO) *δ* 10.54 (s, 1H), 8.66 (d, *J* = 2.0 Hz, 1H), 7.81 (dd, *J* = 2.0 Hz, 8.8 Hz, 1H), 7.72 (s, 1H), 7.65 (d, *J =* 7.6 Hz, 1H), 7.24-7.27 (m, 2H), 7.15-7.19 (m, 1H), 7.07 (d, *J* = 8.8 Hz, 1H), 6.94-6.98 (m, 1H), 6.85-6.89 (m, 2H), 6.81 (d, *J =* 7.6 Hz, 1H), 4.21 (t*, J =* 7.2 Hz, 2H), 3.82 (s, 3H), 3.72 (s, 3H), 3.00 (t, *J =* 6.8 Hz, 2H).

### Example 22

This example provides Compound M22. With Compound d-1 (80 mg, 0.28 mmol) and Compound e-9 (104 mg, 0.38 mmol) as raw materials, Compound M22 was prepared in the same method as Compound M1, and 85 mg of Compound M22 was given as a pale yellow solid, with a yield of 61.6%. The reaction formula of Compound M22 is shown below:

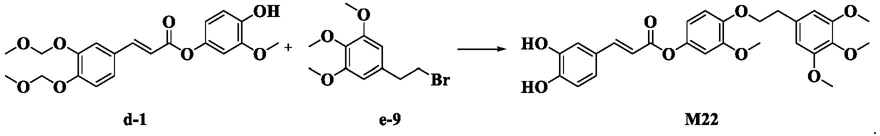

¹H NMR (400 MHz, CDCl₃) *δ* 7.64 (d, *J* = 15.4 Hz, 1H), 7.50 (d, *J* = 7.9 Hz, 2H), 7.35 (s, 1H), 7.25 - 7.11 (m, 3H), 6.89 (d, *J* = 8.8 Hz, 2H), 6.50 (s, 2H), 6.40 (d, *J =* 15.4 Hz, 1H), 5.27 (s, 4H), 4.17 (t, *J=* 7.0 Hz, 2H), 3.86 (s, 6H), 3.84 (s, 3H), 3.54 (s, 3H), 3.52 (s, 3H), 3.01 (t, *J* = 7.0 Hz, 2H).

### Example 23

This example provides Compound M23. With Compound d-3 (150 mg, 0.42 mmol) and Compound e-10 (34 mg, 0.23 mmol) as raw materials, Compound M23 was prepared in the same method as Compound M1, and 80 mg of Compound M23 was given as a pale yellow solid, with a yield of 83.3%. The reaction formula of Compound M23 is shown below:

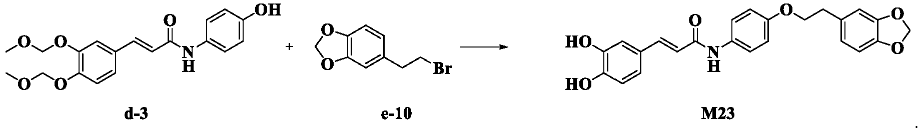

¹H NMR (400 MHz, CDCl₃) *δ* 10.42 (s, 1H), 8.65-8.66 (m, 1H), 7.81-7.83 (m, 1H), 7.69 (s, 1H), 7.47-7.49 (m, 1H), 7.24-7.27 (m, 2H), 7.06-7.08 (m, 1H), 6.96-6.99 (m, 1H), 6.86-6.88 (m, 2H), 6.69-6.71 (m, 1H), 5.80 (s, 2H), 4.21-4.22 (m, 2H), 3.01 (t, J = 6.8 Hz, 2H).

### Example 24

This example provides Compound M24. With Compound d-2 (80 mg, 0.27 mmol) and Compound e-1 (80 mg, 0.33 mmol) as raw materials, Compound M24 was prepared in the same method as Compound M1, and 84 mg of Compound M24 was given as a pale yellow solid, with a yield of 66.7%. The reaction formula of Compound M24 is shown below:

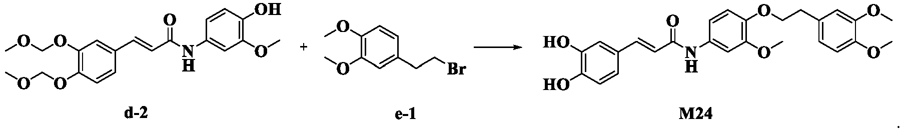

¹H NMR (400 MHz, Acetone-*d*₆) *δ* 8.95 (s, 1H), 8.25 (s, 1H), 8.00 (s, 1H), 7.60 (d, *J* = 8.6 Hz, 2H), 7.48 (d, *J =* 15.6 Hz, 1H), 7.06 (d, *J* = 2.4 Hz, 1H), 6.95 (d, *J =* 9.2 Hz, 2H), 6.89 - 6.78 (m, 4H), 6.54 (d, *J =* 15.5 Hz, 1H), 4.13 (t, *J* = 7.0 Hz, 2H), 3.84 (s, 3H), 2.97 (t, *J =* 7.0 Hz, 2H).

### Example 25

This example provides Compound M25. With Compound d-2 (80 mg, 0.27 mmol) and Compound e-4 (64 mg, 0.35 mmol) as raw materials, Compound M25 was prepared in the same method as Compound M1, and 80 mg of Compound M25 was given as a pale yellow solid, with a yield of 73.4%. The reaction formula of Compound M25 is shown below:

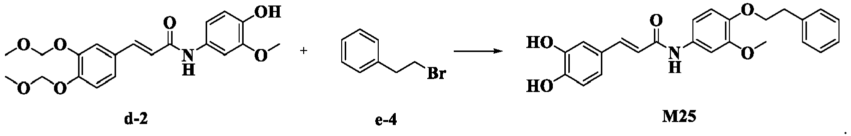

¹H NMR (400 MHz, Acetone-*d*₆) *δ* 7.60 (d, *J =* 9.1 Hz, 2H), 7.50 (d, *J* = 15.5 Hz, 1H), 7.39 - 7.25 (m, 4H), 7.14 (t, *J* = 6.3 Hz, 1H), 7.03 (d, *J* = 2.1 Hz, 1H), 6.95 (dd, *J* = 8.2, 2.1 Hz, 1H), 6.89 - 6.76 (m, 3H), 6.54 (d, *J =* 15.5 Hz, 1H), 4.16 (t, *J =* 6.9 Hz, 2H), 3.84 (s, 3H), 3.02 (t, *J =* 6.9 Hz, 3H).

### Example 26

This example provides Compound M26. With Compound f-1 (100 mg, 0.67 mmol) and Compound g-1 (233 mg, 0.77 mmol) as raw materials, Compound M26 was prepared in the same method as Compound M1, without the step of acidification, and 200 mg of Compound M26 was given as a pale yellow solid, with a yield of 69%. The reaction formula of Compound M26 is shown below:

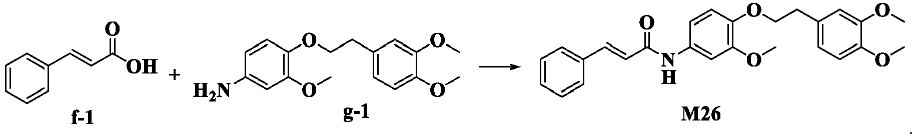

¹H NMR (400 MHz, CDCl₃) *δ* 8.00 (s, 1H), 7.53 (d, *J* = 8.6 Hz, 2H), 7.42 (d, *J* = 15.6 Hz, 1H), 7.23-7.26 (m, 2H), 7.06 (d, *J* = 2.4 Hz, 1H), 6.95 (d, *J* = 9.2 Hz, 2H), 6.89 - 6.78 (m, 4H), 6.54 (d, *J =* 15.5 Hz, 1H), 4.12 (t, *J =* 7.0 Hz, 2H), 3.93 (s, 3H), 3.92 (s, 3H), 3.86 (s, 3H), 2.99 (t, *J =* 7.0 Hz, 2H).

### Example 27

This example provides Compound M27. With Compound f-2 (100 mg, 0.56 mmol) and Compound g-1 (233 mg, 0.77 mmol) as raw materials, Compound M27 was prepared in the same method as Compound M26, without the step of acidification, and 106 mg of Compound M27 was given as a pale yellow solid, with a yield of 40.8%. The reaction formula of Compound M27 is shown below:

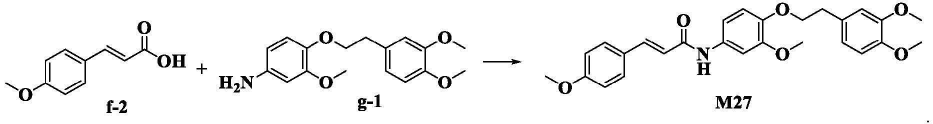

¹H NMR (400 MHz, CDCl₃) *δ* 7.23 (s, 1H), 7.53 (d, *J =* 8.6 Hz, 2H), 7.38 (d, *J =* 15.6 Hz, 1H), 7.23-7.26 (m, 1H), 7.06 (s, 1H), 6.95 (d, *J =* 9.2 Hz, 2H), 6.89 - 6.78 (m, 4H), 6.54 (d, *J =* 15.5 Hz, 1H), 4.12 (t*, J =* 7.0 Hz, 2H), 3.91 (s, 3H), 3.88 (s, 3H), 3.83 (s, 3H), 2.99 (t, *J=* 7.0 Hz, 2H).

### Example 28

This example provides Compound M28. With Compound f-3 (100 mg, 0.52 mmol) and Compound g-1 (233 mg, 0.77 mmol) as raw materials, Compound M28 was prepared in the same method as Compound M26, without the step of acidification, and 180 mg of Compound M28 was given as a pale yellow solid, with a yield of 72.6%. The reaction formula of Compound M28 is shown below:

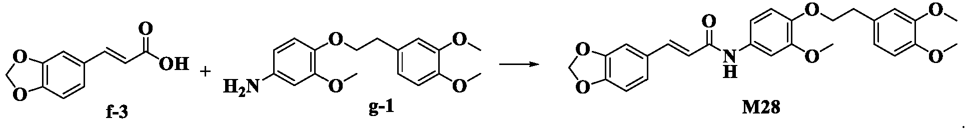

¹H NMR (400 MHz, CDCl₃) *δ* 8.60 (m, 1H), 7.80 (m, 1H), 7.66 (s, 1H), 7.47-7.49 (m, 1H), 7.24-7.27 (m, 2H), 7.06-7.08 (m, 1H), 6.96-6.99 (m, 1H), 6.86-6.88 (m, 2H), 6.69-6.71 (m, 1H), 5.77 (s, 2H), 3.91 (s, 3H), 3.88 (s, 3H), 3.83 (s, 3H), 4.21-4.22 (m, 2H), 2.99 (t, *J =* 6.8 Hz, 2H).

### Example 29

This example provides Compound M29. With Compound f-4 (100 mg, 0.60 mmol) and Compound g-1 (233 mg, 0.77 mmol) as raw materials, Compound M29 was prepared in the same method as Compound M26, without the step of acidification, and 150 mg of Compound M29 was given as a pale yellow solid, with a yield of 55.6%. The reaction formula of Compound M29 is shown below:

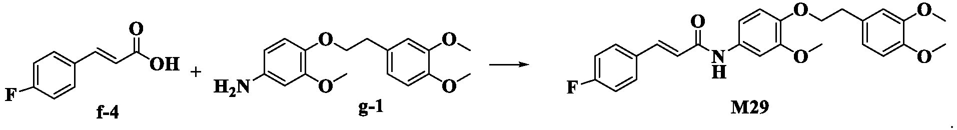

¹H NMR (400 MHz, CDCl₃) *δ* 7.77 (m, 1H), 7.66 (s, 1H), 7.47-7.49 (m, 2H), 7.24-7.27 (m, 2H), 7.06-7.08 (m, 2H), 6.96-6.99 (m, 1H), 6.86-6.88 (m, 2H), 6.69-6.71 (m, 1H), 3.89 (s, 3H), 3.86 (s, 3H), 3.80 (s, 3H), 4.21-4.22 (m, 2H), 2.99 (t, *J =* 6.8 Hz, 2H).

### Example 30

This example provides Compound M30. With Compound f-5 (100 mg, 0.62 mmol) and Compound g-1 (233 mg, 0.77 mmol) as raw materials, Compound M30 was prepared in the same method as Compound M26, without the step of acidification, and 150 mg of Compound M30 was given as a pale yellow solid, with a yield of 54.2%. The reaction formula of Compound M30 is shown below:

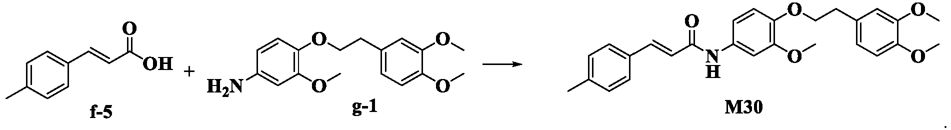

¹H NMR (400 MHz, CDCl₃) *δ* 7.88 (m, 1H), 7.49 (s, 1H), 7.47 (m, 2H), 7.20 (m, 2H), 7.06-7.08 (m, 2H), 6.99 (m, 1H), 6.86-6.88 (m, 2H), 6.69-6.71 (m, 1H), 3.89 (s, 3H), 3.86 (s, 3H), 3.80 (s, 3H), 3.72 (s, 3H), 4.23 (m, 2H), 2.969 (t, *J* = 6.8 Hz, 2H).

### Example 31

This example provides Compound M31. With Compound f-6 (100 mg, 0.55 mmol) and Compound g-1 (233 mg, 0.77 mmol) as raw materials, Compound M31 was prepared in the same method as Compound M26, without the step of acidification, and 116 mg of Compound M31 was given as a pale yellow solid, with a yield of 45%. The reaction formula of Compound M31 is shown below:

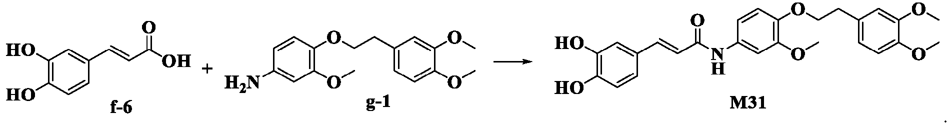

HR-MS (ESI) calcd for C₂₆H₂₈O₆N₂ (M+H)⁺: 464.1730, found 464.1710.

### Pharmacological experiments

### Experimental example 1

### IC₅₀ measurement of phenyl acrylic acid derivatives for ACAT1 targets

### (1) Experimental method

### Screening method of ACAT1 small molecule inhibitors

ACAT1 can catalyze two molecules of acetyl coenzyme A to reversibly form acetoacetyl coenzyme A. Its activity was measured through the reaction between ACAT1-catalyzed substrate acetoacetyl-coenzyme A and coenzyme A, from which the product acetyl-coenzyme A is produced. Since acetoacetyl-coenzyme A is specifically absorbed in a specific spectrum, the impact on the enzyme activity of ACAT1 can be reflected through the detection of an increase or decrease of absorption in a specific spectrum.

ACAT1 recombinant proteins were expressed and purified by *Escherichia coli,* and the concentration of ACAT1 recombinant proteins obtained was 1 mg/mL. The buffers adopted for ACAT1 small molecule inhibitor screening were 50 mM Tris-HCl (pH 8.1), 20 mM MgCl2, and 40 mM KCl. In 200 µL of enzyme catalytic system, 1 µL of ACAT1 recombinant proteins, the substrate acetyl-coenzyme A whose final concentration was changed from 25 µM to 100 µM through a CPM probe, and small molecule inhibitors at different concentrations were added, respectively. The measurement was carried out through a microplate reader (Biotek Synergy H1) and using excitation light at 355 nm and emission light at 460 nm, and the IC₅₀ of the inhibitors was calculated through software Prism 7.0.

### (2) Experimental result

The screening results of the impact on the activity of ACAT1 *in vitro* are shown in Table 1. The experimental results showed that Compounds 1, 5, 7, 9, 10, 12 and 17 had strong inhibitory activity against ACAT1.

**Table 1**

| Compound | ACAT1 | Compound | ACAT1 |
|---|---|---|---|
| | IC₅₀ (µmol/L) | | IC₅₀ (µmol/L) |
| M1 | 84.4 | M10 | 55 |
| M2 | >100 | M11 | 136 |
| M3 | >100 | M12 | 59 |
| M4 | 112 | M13 | 152 |
| M5 | 87 | M14 | 111 |
| M6 | 134 | M15 | 312 |
| M7 | 91 | M16 | 172 |
| M8 | 114 | M17 | 60 |
| M9 | 66 | | |

As seen from the data in Table 1, Compounds M1, M5, M7, M9, M10, M12 and M17 had strong inhibitory activities against ACAT1, and their IC50 values were all below 100 µmol/L.

### Experimental example 2

### Impact of phenyl acrylic acid derivatives on the activity of pyruvate kinase

### (1) Experimental method

### Screening method for small molecule modulators of pyruvate kinase

Principle: In the presence of adenosine diphosphate (ADP), pyruvate kinase (PK) catalyzed the conversion of phosphoenolpyruvate (PEP) to pyruvate; in the presence of reduced coenzyme I (NADH), pyruvate was converted to lactate by LDH, and if NADH was labelled with fluorescence, the fluorescence-labelled NADH was changed to fluorescent NAD.

Two recombinant protein pyruvate kinase isoforms: PKLR (15501-H07E) and PKM2 (11430-H07E) were purchased from Beijing Tsingke Biotech Co., Ltd. The buffers adopted for PK activity screening were 100 mM Tris-HCl (pH 8.0), 100 mM KCl, and 10 mM MgCl₂. In 200 µL of enzyme catalytic system, various substrates and enzymes were added to the final concentration of ADP (0.6 mM), PEP (0.5 mM), NADH (180 mM), FBP (10 mM) and LDH (8 units), respectively, and small molecule inhibitors at different concentrations were added. The measurement was carried out by detecting the change in the light absorption at 340 nm through a microplate reader (Biotek Synergy H1), and the inhibitory or activating activity was calculated through software Prism 7.0.

### (2) Experimental result

The impact of compounds on the activity of pyruvate kinase *in vitro* is shown in Table 2.

**Table 2**

| Compound | PKLR (OD value) | PKM2 (OD value) |
|---|---|---|
| M1 | 0.957247 | 0.886667 |
| M2 | 1.264338 | 1.21125 |
| M3 | 0.951512 | 0.872917 |

As can be seen from the data in Table 2, Compounds M1 and M3 had certain inhibitory activity on PKM2, while Compound M2 had a certain activating effect on both PKLR and PKM2.

The applicant has stated that although the phenyl acrylic acid compound, the preparation method therefor and the use thereof in the present application are described through the embodiments described above, the present application is not limited to the embodiments described above, which means that implementation of the present application does not necessarily depend on the embodiments described above. It is to be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

## Claims

1. A phenyl acrylic acid compound, having a structure represented by Formula I: 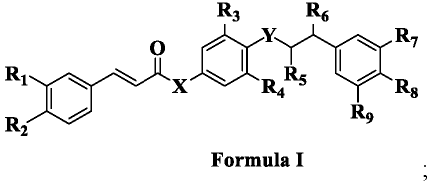
wherein R₁, R₂, R₃, R₄, R₇, R₈ and R₉ are each independently selected from any one of hydrogen, halogen, hydroxyl, dimethylamino, cyano, nitro, methylamino, methylsulfonyl, dimethylsulfamoyl, amino, carboxyl, C1-C6 alkoxycarbonyl, C1-C6 alkylcarbonyloxy, C1-C6 alkyl, C1-C6 alkoxy, C1-C6 alkoxymethyleneoxy, C1-C6 alkanoyl, C1-C6 trihaloalkyl or C1-C6 trihaloalkoxy; or, R₁, R₂, R₇, R₈ and R₉ are each independently selected from -O(CH₂)nO- and joined to substituted phenyl to form a ring, wherein n is selected from 1, 2 or 3;
R₅ is selected from any one of hydrogen, amino, C1-C6 alkyl, C1-C6 alkoxymethyl or C1-C6 alkylamino;
R₆ is selected from any one of hydrogen, hydroxyl, amino, carbonyl, C1-C6 alkylamino, C1-C6 alkylcarbonyloxy or C1-C6 alkoxycarbonyl;
X is selected from O or NH; and
Y is selected from any one of O, NH, S, sulfoxide or sulfone.

2. The phenyl acrylic acid compound according to claim 1, wherein the phenyl acrylic acid compound is selected from any one of Formula II to Formula V: 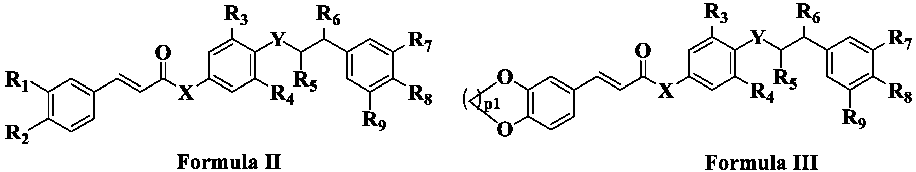 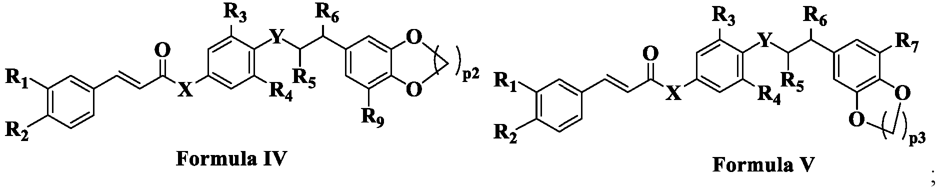
wherein R₁ and R₂ are each independently selected from any one of hydrogen, fluorine, chlorine, bromine, hydroxyl, dimethylamino, cyano, nitro, methylamino, methylsulfonyl, dimethylsulfamoyl, amino, carboxyl, C1-C4 alkoxycarbonyl, C1-C4 alkylcarbonyloxy, C1-C4 alkyl, C1-C4 alkoxy, C1-C4 alkoxymethyleneoxy, C1-C4 trihaloalkyl or C1-C4 trihaloalkoxy;
R₃ and R₄ are each independently selected from any one of hydrogen, fluorine, chlorine, bromine, hydroxyl, dimethylamino, cyano, nitro, methylamino, methylsulfonyl, dimethylsulfamoyl, amino, carboxyl, C1-C4 alkoxycarbonyl, C1-C4 alkylcarbonyloxy, C1-C4 alkyl, C1-C4 alkoxy, C1-C4 trihaloalkyl or C1-C4 trihaloalkoxy;
R₅ is selected from any one of hydrogen, amino, C1-C4 alkyl, C1-C4 alkoxymethyl or C1-C4 alkylamino;
R₆ is selected from any one of hydrogen, hydroxyl, amino, carbonyl, C1-C4 alkylamino, C1-C4 alkylcarbonyloxy or C1-C4 alkoxycarbonyl;
R₇, R₈ and R₉ are each independently selected from any one of hydrogen, fluorine, chlorine, bromine, hydroxyl, dimethylamino, cyano, nitro, methylamino, methylsulfonyl, dimethylsulfamoyl, amino, carboxyl, C1-C4 alkyl, C1-C4 alkoxy, C1-C4 alkoxycarbonyl, C1-C4 alkylcarbonyloxy, C1-C3 alkanoyl, C1-C4 alkoxymethyleneoxy, C1-C4 trihaloalkyl or C1-C4 trihaloalkoxy;
X is selected from O or NH;
Y is selected from any one of O, NH, S, sulfoxide or sulfone; and
p1, p2 and p3 are each independently selected from 1, 2 or 3.

3. The phenyl acrylic acid compound according to claim 2, wherein R₁ and R₂ are each independently selected from any one of hydrogen, fluorine, chlorine, bromine, hydroxyl, dimethylamino, cyano, nitro, methoxycarbonyl, ethoxycarbonyl, methylamino, methylsulfonyl, dimethylsulfamoyl, amino, methyl, ethyl, methoxy, ethoxy, C1-C2 alkylcarbonyloxy, C1-C2 alkoxymethyleneoxy, C1-C2 trihaloalkyl or C1-C2 trihaloalkoxy;
R₃ and R₄ are each independently selected from any one of hydrogen, fluorine, chlorine, bromine, hydroxyl, dimethylamino, cyano, nitro, methoxycarbonyl, ethoxycarbonyl, methylamino, methylsulfonyl, dimethylsulfamoyl, amino, methyl, ethyl, methoxy, ethoxy, C1-C2 alkylcarbonyloxy, C1-C2 trihaloalkyl or C1-C2 trihaloalkoxy;
R₅ is selected from any one of hydrogen, amino, methyl, ethyl, C1-C2 alkoxymethyl or C1-C2 alkylamino;
R₆ is selected from any one of hydrogen, amino, hydroxyl, carbonyl, C1-C2 alkylamino, C1-C2 alkylcarbonyloxy or C1-C2 alkoxycarbonyl;
R₇, R₈ and R₉ are each independently selected from any one of hydrogen, fluorine, chlorine, bromine, hydroxyl, dimethylamino, cyano, nitro, methylamino, methylsulfonyl, dimethylsulfamoyl, amino, carboxyl, methyl, ethyl, methoxy, ethoxy, methoxycarbonyl, ethoxycarbonyl, formyl, acetyl, propionyl, butyryl, pentanoyl, isobutyryl, 2-methylbutanoyl, C1-C2 alkoxymethyleneoxy, C1-C2 trihaloalkyl or C1-C2 trihaloalkoxy;
X is selected from O or NH;
Y is selected from any one of O, NH, S, sulfoxide or sulfone; and
p1, p2 and p3 are each independently selected from 1 or 2.

4. The phenyl acrylic acid compound according to claim 2 or 3, wherein R₁ and R₂ are each independently selected from any one of hydrogen, fluorine, chlorine, bromine, hydroxyl, dimethylamino, cyano, nitro, methoxycarbonyl, methylamino, methylsulfonyl, dimethylsulfamoyl, amino, methyl, ethyl, methoxy, ethoxy, trifluoromethyl, trifluoromethoxy or methoxymethyleneoxy;
R₃ and R₄ are each independently selected from any one of hydrogen, fluorine, chlorine, bromine, hydroxyl, dimethylamino, cyano, nitro, methoxycarbonyl, methylamino, methylsulfonyl, dimethylsulfamoyl, amino, methyl, ethyl, methoxy, ethoxy, trifluoromethyl or trifluoromethoxy;
R₅ is selected from any one of hydrogen, methyl, ethyl, methoxymethyl or amino;
R₆ is selected from any one of hydrogen, hydroxyl, amino, carbonyl or C1-C2 alkylcarbonyloxy;
R₇, R₈ and R₉ are each independently selected from any one of hydrogen, fluorine, chlorine, bromine, hydroxyl, dimethylamino, cyano, nitro, methylamino, methylsulfonyl, dimethylsulfamoyl, amino, carboxyl, methyl, ethyl, methoxy, ethoxy, trifluoromethyl, trifluoromethoxy, methoxycarbonyl, formyl, acetyl, propionyl, butyryl, pentanoyl, isobutyryl or methoxymethyleneoxy;
X is selected from O or NH;
Y is selected from any one or a combination of at least two of O, NH, S, sulfoxide or sulfone; and
p1, p2 and p3 are each independently selected from 1.

5. The phenyl acrylic acid compound according to any one of claims 1 to 4, wherein the phenyl acrylic acid compound is selected from any one of M1 to M31: 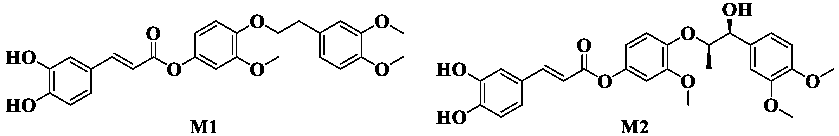 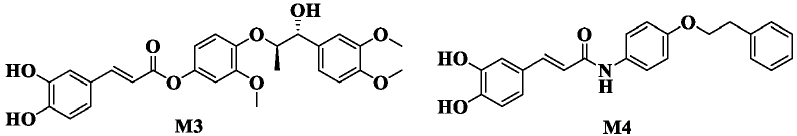 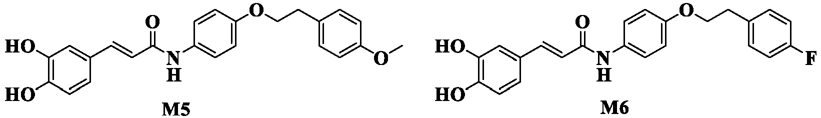 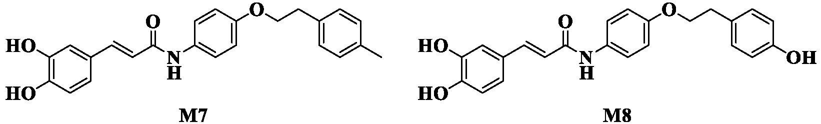 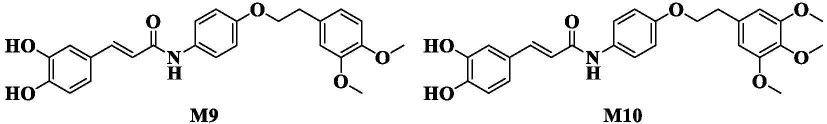 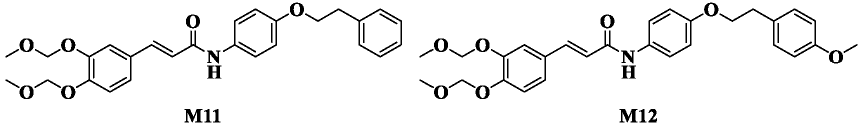 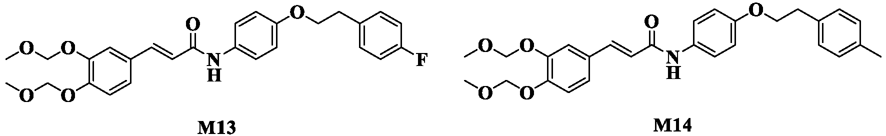 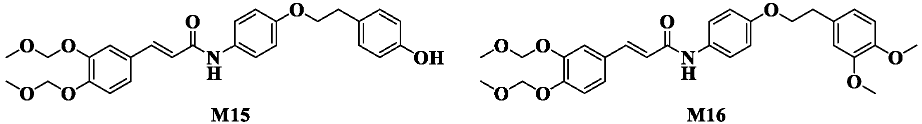 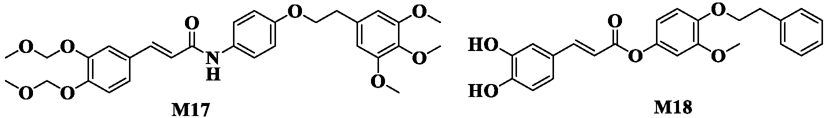 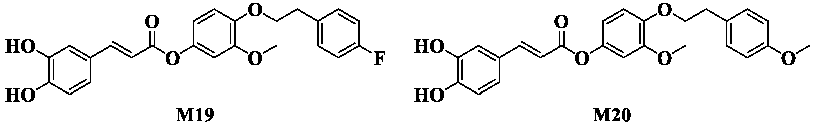 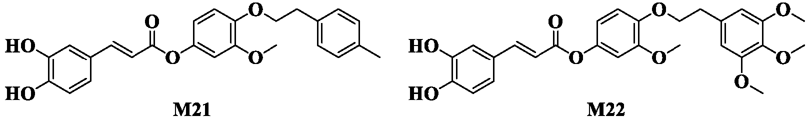 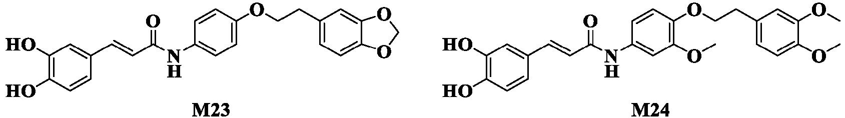 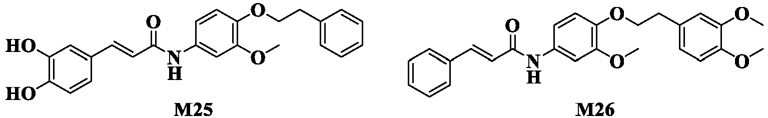 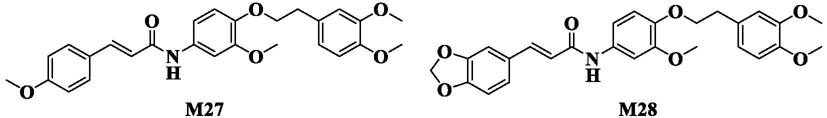 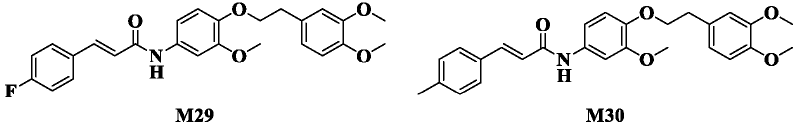 or

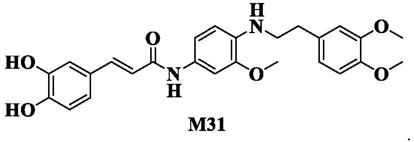

6. A tautomer, enantiomer or diastereomer of the phenyl acrylic acid compound according to any one of claims 1 to 5, or a pharmaceutically acceptable salt thereof;
preferably, the pharmaceutically acceptable salt comprises any one or a combination of at least two of hydrochloride, hydrobromide, phosphate, sulphate, methanesulfonate, p-toluenesulfonate, acetate, trifluoroacetate, salicylate, amino acid salt, 2-O-β-D-glucopyranosyl-L-ascorbic acid salt, maleate, tartrate, fumarate, citrate, lactate, a sodium salt, a potassium salt, a calcium salt, a magnesium salt, a lithium salt, an ammonium salt, or a salt of an organic base capable of providing a physiologically acceptable cation;
preferably, the salt of an organic base capable of providing a physiologically acceptable cation comprises any one or a combination of at least two of a methylamine salt, a dimethylamine salt, a trimethylamine salt, a piperidine salt, a morpholine salt or a tris(2-hydroxyethyl)amine salt.

7. A method for preparing the phenyl acrylic acid compound according to any one of claims 1 to 5, comprising the following steps:
(1) carrying out a condensation reaction on a compound represented by Formula a and a compound represented by Formula b to obtain a compound represented by Formula c, wherein the reaction formula is shown below: 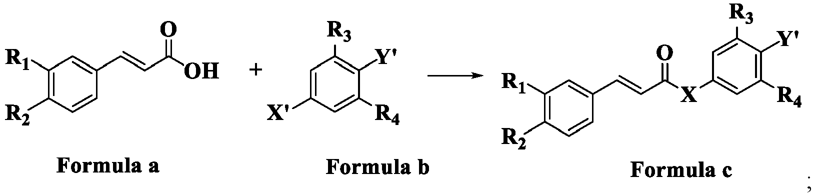
(2) carrying out deprotection on the compound represented by Formula c to obtain a compound represented by Formula d, wherein the reaction formula is shown below: 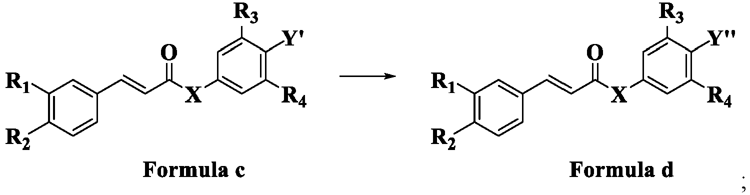
(3) carrying out a substitution reaction on the compound represented by Formula d and a compound represented by Formula e to obtain a compound represented by Formula I, wherein the reaction formula is shown below: 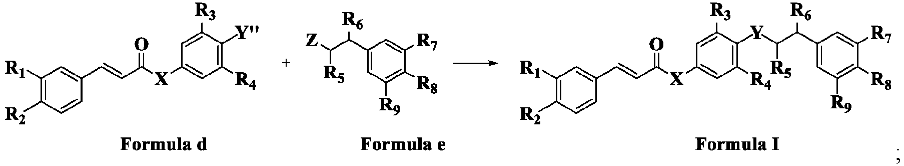
or the preparation method comprises the following step: carrying out a condensation reaction on a compound represented by Formula f and a compound represented by Formula g to obtain a compound represented by Formula I, wherein the reaction formula is shown below: 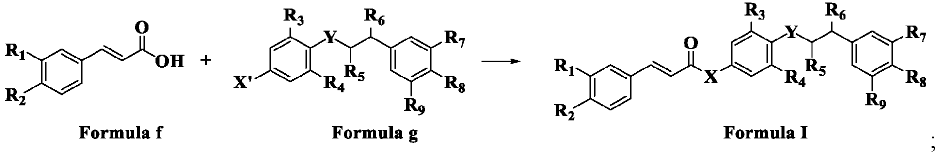
wherein X' is selected from OH or NH₂;
Y' is selected from any one of tert-butyldimethylsilyloxy, tert-butyldiphenylsilyloxy, trimethylsilyloxy, triethylsilyloxy, benzyloxy, p-methoxybenzyloxy, methoxymethyleneoxy, benzyloxycarbonyloxy or tert-butoxycarbonyloxy;
Y" is selected from any one of OH, NH₂ or SH;
Z is selected from any one of hydroxyl, chlorine, bromine, iodine, p-toluenesulfonyl or methylsulfonyl.

8. The preparation method according to claim 7, wherein in step (1), the condensation reaction is carried out under an alkaline condition;
preferably, in step (1), the condensation reaction is carried out in the presence of a condensating agent, and the condensating agent comprises EDCI and/or DMAP;
preferably, in step (1), the condensation reaction is carried out at a temperature of 0 °C to 30 °C for 5 hours to 15 hours;
preferably, in step (1), a molar ratio of the compound represented by Formula a to the compound represented by Formula b is 1:(0.8-1.5);
preferably, in step (2), the deprotection is carried out via a hydrolysis reaction or a hydrogenation reaction;
preferably, in step (2), the deprotection is carried out via a hydrolysis reaction, and the hydrolysis reaction is carried out under an acidic condition or an alkaline condition;
preferably, in step (3), the substitution reaction is carried out under an alkaline condition;
preferably, in step (3), the substitution reaction is carried out at a temperature of 60 °C to 100 °C for 2 hours to 6 hours;
preferably, in step (3), a molar ratio of the compound represented by Formula d to the compound represented by Formula e is 1:(1-2);
preferably, the condensation reaction is carried out on the compound represented by Formula f and the compound represented by Formula g under an alkaline condition;
preferably, the condensation reaction is carried out on the compound represented by Formula f and the compound represented by Formula g in the presence of a condensating agent, and the condensating agent comprises EDCI and/or DMAP;
preferably, a molar ratio of the compound represented by Formula f to the compound represented by Formula g is 1:(0.8-1.5).

9. A pharmaceutical composition, comprising an active ingredient and a pharmacodynamically acceptable carrier, wherein the active ingredient comprises the phenyl acrylic acid compound according to any one of claims 1 to 5 or the tautomer, enantiomer or diastereomer of the phenyl acrylic acid compound or the pharmaceutically acceptable salt thereof according to claim 6;
preferably, a mass percentage of the active ingredient in the pharmaceutical composition is 0.1% to 95%.

10. Use of the phenyl acrylic acid compound according to any one of claims 1 to 5, the tautomer, enantiomer or diastereomer of the phenyl acrylic acid compound or the pharmaceutically acceptable salt thereof according to claim 6 or the pharmaceutical composition according to claim 9 in the preparation of a drug for preventing or treating a tumor, an autoimmune disease, an inflammatory disease, a neurodegenerative disease or aging;
preferably, the tumor is selected from any one or a combination of at least two of glioma, melanoma, gastric cancer, lung cancer, breast cancer, kidney cancer, liver cancer, oral epithelial carcinoma, head and neck tumor, cervical cancer, ovarian cancer, pancreatic cancer, prostate cancer, colon cancer, rectal adenocarcinoma, leukemia or lymphoma;
preferably, the autoimmune disease comprises any one or a combination of at least two of rheumatoid arthritis, systemic lupus erythematosus, ulcerative colitis, psoriasis, dermatitis or lateral sclerosis of the spinal cord;
preferably, the inflammatory disease comprises any one or a combination of at least two of polyarteritis, phlebitis or reflux esophagitis;
preferably, the neurodegenerative disease comprises senile dementia and/or Parkinson's disease.
